(19) **Europäisches Patentamt / European Patent Office / Office européen des brevets**

(11) **EP 4 710 903 A1**

(12) **EUROPEAN PATENT APPLICATION**
published in accordance with Art. 153(4) EPC

(43) Date of publication:
**18.03.2026 Bulletin 2026/12**

(21) Application number: **24811122.1**

(22) Date of filing: **21.05.2024**

(51) International Patent Classification (IPC):
*A61F 13/42* (2006.01)    *A61F 13/84* (2006.01)

(52) Cooperative Patent Classification (CPC):
**A61F 13/42; A61F 13/511; A61F 13/53; A61F 13/84**

(86) International application number:
**PCT/JP2024/018735**

(87) International publication number:
**WO 2024/242116 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.05.2023 JP 2023083677**

(71) Applicant: **Unicharm Corporation**
**Shikokuchuo-shi, Ehime 799-0111 (JP)**

(72) Inventors:
• **UEDA, Takahiro**
**Kanonji-shi, Kagawa 769-1602 (JP)**
• **NISHIMURA, Kiyoko**
**Kanonji-shi, Kagawa 769-1602 (JP)**
• **YOSHIMASA, Wataru**
**Kanonji-shi, Kagawa 769-1602 (JP)**
• **GODA, Hiroki**
**Kanonji-shi, Kagawa 769-1602 (JP)**
• **NOMOTO, Takashi**
**Kanonji-shi, Kagawa 769-1602 (JP)**
• **HASHINO, Akira**
**Kanonji-shi, Kagawa 769-1602 (JP)**
• **SUZUKI, Yuya**
**Kanonji-shi, Kagawa 769-1602 (JP)**

(74) Representative: **Dolleymores**
**9 Rickmansworth Road**
**Watford, Hertfordshire WD18 0JU (GB)**

(54) **ABSORBENT ARTICLE**

(57) The present invention provides an absorbent article with which the wearer can easily grasp the testing result. An absorbent article (1) according to one aspect includes a testing member (60) for testing a health condition of the wearer. The testing member (60) includes an indicator portion (62) that develops color based on an excreta component as a component contained in excreta. The absorbent article (1) includes a first hydrophobic part (71) having hydrophobicity that covers a skin side (T1) of the indicator portion (62) of the testing member (60). The indicator portion (62) after color development is visually recognizable from the skin side of the absorbent article.

FIG. 3

EP 4 710 903 A1

**Description**

[TECHNICAL FIELD]

The present invention relates to an absorbent article including a testing member for testing a health condition of a wearer.

[BACKGROUND ART]

Patent Literature 1 discloses an absorbent article including a testing member for testing a health condition of a wearer. The testing member includes at least an indicator portion that develops color based on a component contained in excreta (hereinafter, an excreta component).

[CITATION LIST]

[PATENT LITERATURE]

[PATENT LITERATURE 1] Japanese Patent Laid-Open No. 2020-022585

[SUMMARY OF INVENTION]

The testing member in Patent Literature 1 is configured to be seen from a non-skin side of the absorbent article. Thus, with the absorbent article that is used by being fastened to a wearable article, a testing result is grasped from the non-skin side after once removing the absorbent article from the wearable article or partially peeling off and turning up the absorbent article from the wearable article. With the absorbent article that is used by being worn, a testing result is grasped from the non-skin side after once taking off the absorbent article. Thus, the testing result is difficult to be visually recognized in some cases.
The present invention is made in view of the disadvantages described above, and provides an absorbent article with which the wearer can easily grasp the testing result.
An absorbent article according to one aspect includes a testing member for testing a health condition of the wearer. The testing member includes an indicator portion that develops color based on an excreta component as a component contained in excreta. The absorbent article includes a first hydrophobic part having hydrophobicity that covers a skin side of the indicator portion of the testing member. The indicator portion after color development is visually recognizable from the skin side of the absorbent article.
The absorbent article according to another aspect includes the testing member for testing a health condition of the wearer, and an absorbent core. The testing member includes an indicator portion that develops color based on an excreta component as a component contained in excreta. The absorbent article includes a second hydrophobic part having hydrophobicity that covers the non-skin side of the testing member. The absorbent core is arranged on the non-skin side relative to the second hydrophobic part. The indicator portion after color development is visually recognizable from the skin side of the absorbent article.

[Brief Description of Drawings]

[FIG. 1] FIG. 1 is a plan view of an absorbent article according to an embodiment as seen from a skin side.

[FIG. 2] FIG. 2 is a plan view of the absorbent article according to the embodiment as seen from a non-skin side.

[FIG. 3] FIG. 3 is a cross-sectional view taken along line A-A indicated in FIG. 1.

[FIG. 4] FIG. 4 is a cross-sectional view taken along line B-B indicated in FIG. 1.

[FIG. 5] FIG. 5 is a cross-sectional view taken along line C-C indicated in FIG. 1.

[FIG. 6] FIG. 6 is an enlarged view of a portion D indicated in FIG. 5.

[FIG. 7] FIG. 7 is a cross-sectional photograph of a cross section taken along a width direction of a testing member.

[FIG. 8] FIG. 8 is a cross-sectional view taken along the width direction of the testing member.

[FIG. 9] FIG. 9 is a diagram for describing a color developing aspect of the testing member.

[FIG. 10] FIG. 10 is a diagram schematically illustrating a cross section along the width direction in a state of wearing a wearable article to which the absorbent article is attached.

[FIG. 11] FIG. 11 is a front view of a container according to a first mode.

[FIG. 12] FIG. 12 is a back view of the container according to the first mode.

[FIG. 13] FIG. 13 is a diagram illustrating a container according to a second mode.

[FIG. 14] FIG. 14 is a diagram for describing a permeation test for artificial body fluid.

[DESCRIPTION OF EMBODIMENTS]

(1) Outline of embodiment

From the description in the present specification and the attached drawings, at least the following matters become apparent.
The invention according to a first aspect is an absorbent article. An absorbent article includes a testing member for testing a health condition of the wearer. The testing member includes an indicator portion that develops color based on an excreta component as a component contained in excreta. The absorbent article includes a first hydrophobic part having hydrophobicity that covers a skin side of the indicator portion of the testing member. The indicator portion after color development is visually recognizable from the skin side of the absorbent article. According to the present aspect, the indicator portion after color development is visually recognizable from the skin side of the absorbent article, so that the wearer can easily grasp a testing result by seeing the absorbent article from the skin side of the absorbent article without peeling off the absorbent article from the wearable article or taking off the absorbent article from the wearable article. The first hydrophobic part covers the skin side of the indicator portion, so that it is possible to prevent excreta from directly reaching the indicator portion from the skin side of the indicator portion. Thus, it is possible to prevent visibility of the indicator portion from being lowered due to excreta that has directly reached the indicator portion.
According to a preferred aspect, the invention according to a second aspect may have the following characteristic in the invention according to the first aspect. The testing member includes a contact portion that comes into contact with excreta, and a movement portion that guides, to the indicator portion, an excreta component as a component contained in excreta guided from the contact portion. According to the present aspect, the skin side of the movement portion is covered by the first hydrophobic part, so that it is difficult for excreta to directly reach the indicator portion, and testing accuracy can be improved. The first hydrophobic part covers the skin side of the movement portion. When testing accuracy is improved, a correct testing result can be quickly indicated, and a user can easily grasp the testing result.
According to a preferred aspect, the invention according to a third aspect may have the following characteristic in the invention according to the second aspect. The first hydrophobic part is constituted of a liquid-impermeable skin side sheet. The skin side sheet is arranged on the skin side relative to the testing member, and arranged to straddle the contact portion, the movement portion, and the indicator portion. According to the present aspect, the skin side sheet straddles the skin side of the contact portion, the movement portion, and the indicator portion, so that it is possible to prevent excreta from infiltrating toward the testing member side through a gap between the first hydrophobic parts, and prevent excreta from reaching the testing member via an unintended path.
According to a preferred aspect, the invention according to a fourth aspect may have the following characteristic in the invention according to any one of the first aspect to the third aspect. The absorbent article includes an absorbent core. The first hydrophobic part is constituted of a liquid-impermeable skin side sheet. A visible light transmittance of the skin side sheet is higher than a visible light transmittance of the absorbent core. According to the present aspect, the visible light transmittance of the skin side sheet constituting the first hydrophobic part is relatively high, so that it is possible to prevent visibility of the testing member from being lowered due to the presence of the skin side sheet. By covering at least the indicator portion by the skin side sheet, while preventing the visibility of the testing member from being lowered with a high visible light transmittance, it is possible to prevent visibility of the indicator portion from being lowered due to excreta that has directly reached from the skin side.
According to a preferred aspect, the invention according to a fifth aspect may have the following characteristic in the invention according to any one of the first aspect to the fourth aspect. The absorbent article includes an absorbent core. The testing member is arranged on the skin side relative to the absorbent core. According to the present aspect, the testing member is located on the skin side relative to the absorbent core, and the skin side thereof is not covered by the absorbent core. Thus, it is possible to easily secure visibility of the indicator portion when seen from the skin side of the absorbent article. The user can grasp the testing result more easily.

According to a preferred aspect, the invention according to a sixth aspect may have the following characteristic in the invention according to any one of the first aspect to the fifth aspect. The absorbent article includes a second hydrophobic part having hydrophobicity that covers a non-skin side of the indicator portion of the testing member. The absorbent core is arranged on the non-skin side relative to the second hydrophobic part. According to the present aspect, the testing member is located on the skin side relative to the absorbent core, and the skin side thereof is not covered by the absorbent core. Thus, it is possible to easily secure visibility of the indicator portion when seen from the skin side of the absorbent article. The second hydrophobic part covers the non-skin side of the indicator portion of the testing member, and is arranged between the testing member and the absorbent core, so that it is possible to prevent excreta absorbed by the absorbent core from directly moving to the indicator portion. It is possible to prevent visibility of the indicator portion from being lowered due to excreta absorbed by the absorbent core.

The invention according to a seventh aspect is the absorbent article. The absorbent article according to another aspect includes the testing member for testing a health condition of the wearer, and an absorbent core. The testing member includes an indicator portion that develops color based on an excreta component as a component contained in excreta. The absorbent article includes a second hydrophobic part having hydrophobicity that covers the non-skin side of the testing member. The absorbent core is arranged on the non-skin side relative to the second hydrophobic part. The indicator portion after color development is visually recognizable from the skin side of the absorbent article. According to the present aspect, the testing member is visually recognizable from the skin side of the absorbent article, so that the wearer can easily grasp a testing result by seeing the absorbent article from the skin side of the absorbent article without peeling off the absorbent article from the wearable article or taking off the absorbent article from the wearable article. The testing member is located on the skin side relative to the absorbent core, and the skin side thereof is not covered by the absorbent core. Thus, it is possible to easily secure visibility of the indicator portion when seen from the skin side of the absorbent article. The user can easily grasp the testing result. The second hydrophobic part covers the non-skin side of the indicator portion of the testing member, so that it is possible to prevent excreta absorbed by the absorbent core from moving to the indicator portion. It is possible to prevent visibility of the indicator portion from being lowered due to excreta absorbed by the absorbent core.

According to a preferred aspect, the invention according to an eighth aspect may have the following characteristic in the invention according to the seventh aspect. The testing member includes a contact portion that comes into contact with excreta, and a movement portion that guides, to the indicator portion, an excreta component as a component contained in excreta guided from the contact portion. The second hydrophobic part covers the non-skin side of the contact portion and the non-skin side of the movement portion. According to the present aspect, the second hydrophobic part covers the non-skin side of the contact portion and the non-skin side of the movement portion, so that it is possible to prevent excreta absorbed by the absorbent core from moving to the contact portion and the movement portion. Thus, excreta guided into the contact portion can be assumed to be excreta guided from the skin side of the absorbent article, and an excreta component required for testing can be guided to the movement portion via the contact portion. By guiding the excreta component via an intended path, testing accuracy can be improved, and a correct testing result can be quickly indicated.

According to a preferred aspect, the invention according to a ninth aspect may have the following characteristic in the invention according to any one of the first aspect to the eighth aspect. The testing member is visually recognizable from the skin side of the absorbent article before color development of the indicator portion, and is visually unrecognizable from the non-skin side of the absorbent article. According to the present aspect, the testing member is visually recognizable from the skin side of the absorbent article before color development of the indicator portion, so that it is possible to grasp a position of the testing member of the absorbent article at the time of attachment, and arrange the testing member at an appropriate position. The testing member can be seen from the skin side of the absorbent article, and the testing member cannot be seen from the non-skin side, so that it is possible to intuitively grasp the skin surface and the non-skin surface of the absorbent article depending on whether the testing member is visually recognizable, and improve operability at the time of attachment.

According to a preferred aspect, the invention according to a tenth aspect may have the following characteristic in the invention according to any one of the first aspect to the ninth aspect. The absorbent article includes third hydrophobic parts having hydrophobicity that are arranged on both sides in a width direction of the testing member. A length in the width direction of each of the third hydrophobic parts is longer than a length in the width direction of the testing member. According to the present aspect, the length in the width direction of the third hydrophobic part is longer than the length in the width direction of the testing member, so that it is possible to prevent infiltration of excreta from the lateral side of the testing member over a wide range extending from the testing member toward the outer side in the width direction. Excreta are less likely to be arranged adjacent to the lateral side of the testing member, so that it is possible to prevent visibility of the testing member from being lowered due to excreta accumulated on the lateral side of the testing member.

According to a preferred aspect, the invention according to an eleventh aspect may have the following characteristic in the invention according to any one of the first aspect to the tenth aspect. A first hydrophobic part having hydrophobicity that covers a skin side of the indicator portion of the testing member, and a second hydrophobic part having hydrophobicity that covers a non-skin side of the indicator portion of the testing member. The first hydrophobic part is constituted of a liquid-impermeable skin side sheet. The second hydrophobic part is constituted of a liquid-impermeable non-skin side sheet. The skin side sheet and the non-skin side sheet are in contact with each other at least on an outer side in a width direction relative to the testing member. According to the present aspect, it is possible to prevent infiltration of excreta from the skin side, the non-skin side, and the lateral side of the testing member by the skin side sheet and the non-skin side sheet being in contact with each other, and prevent excreta from reaching the testing member via an unintended path.

According to a preferred aspect, the invention according to a twelfth aspect may have the following characteristic in the invention according to the eleventh aspect. An outer edge of the skin side sheet is displaced from an outer edge of the non-skin side sheet in the width direction. The absorbent article is held between legs, and receives a force from the outer side toward the inner side in the width direction. In this case, the outer edge of the non-skin side sheet is displaced from the outer edge of the skin side sheet in the width direction, and a plurality of deformation base points are disposed at intervals in the width direction, so that the force applied from the outer side in the width direction can be gradually reduced, a shape of the testing member can be maintained, contact with the legs can be alleviated, and a sense of incongruity during wearing can be suppressed.

According to a preferred aspect, the invention according to a thirteenth aspect may have the following characteristic in the invention according to any one of the first aspect to the twelfth aspect. The absorbent article includes a topsheet located on the skin side relative to the testing member, and a backsheet located on the non-skin side relative to the testing member. The topsheet and the backsheet are constituted of sheets containing fibers. An average inter-fiber distance of the backsheet is shorter than an average inter-fiber distance of the topsheet. According to the present aspect, the testing member can be easily seen from the skin side of the absorbent article, and the testing member is difficult to be seen from the non-skin side of the absorbent article. The visibility of the testing member from the skin side of the absorbent article can be easily secured, and operability at the time of attachment can be improved by improving a discrimination property between the skin surface and the non-skin surface of the absorbent article.

According to a preferred aspect, the invention according to a fourteenth aspect may have the following characteristic in the invention according to any one of the first aspect to the thirteenth aspect. The testing member is arranged to be inclined with respect to a front-rear direction of the absorbent article in a plan view of the absorbent article. According to the present aspect, the testing member is inclined with respect to the front-rear direction of the absorbent article, so that the testing member is likely to be more noticeable than outer edges of the other constituent elements (for example, the topsheet and the absorbent core) of the absorbent article, and the user can easily grasp the presence of the testing member.

According to a preferred aspect, the invention according to a fifteenth aspect may have the following characteristic in the invention according to any one of the first aspect to the fourteenth aspect. A projecting part projecting toward an outer side in a width direction of the absorbent article and a recessed part recessed toward an inner side in the width direction with respect to the projecting part are disposed on an outer portion of the testing member. According to the present aspect, the outer portion of the testing member is likely to be noticeable, and the user can easily grasp the presence of the testing member.

According to a preferred aspect, the invention according to a sixteenth aspect may have the following characteristic in the invention according to any one of the first aspect to the fifteenth aspect. The absorbent article includes a topsheet located on a skin side relative to the testing member, and a compressed part obtained by compressing at least the topsheet in a thickness direction. The compressed part includes a first compressed part and a second compressed part arranged on opposite sides across the testing member in a width direction of the absorbent article. A distance in the width direction between the first compressed part and the testing member is different from a distance in the width direction between the second compressed part and the testing member. According to the present aspect, the distance in the width direction between the first compressed part and the testing member is different from the distance in the width direction between the second compressed part and the testing member, and the first compressed part and the second compressed part are asymmetrically arranged with respect to the testing member. Thus, the testing member can be made relatively noticeable with respect to the first compressed part and the second compressed part.

According to a preferred aspect, the invention according to a seventeenth aspect may have the following characteristic in the invention according to any one of the first aspect to the sixteenth aspect. At a cross section along a front-rear direction of the absorbent article, a skin side surface of the testing member is inclined toward the non-skin side from the center in the front-rear direction of the testing member toward an outer end of the testing member. According to the present aspect, the skin side surface is inclined toward the outer side in the front-rear direction of the testing member and toward the non-skin side, so that, when body fluid reaches the skin side surface of the testing member, the body fluid can be guided to the outer side in the front-rear direction of the testing member while being guided to the non-skin side. Thus, the body fluid hardly remains on the testing member, and it is possible to prevent the body fluid from reaching the inside of the testing member via an unintended path.

(2) Absorbent article according to embodiment

The following describes an embodiment of the present invention with reference to the drawings. In the description of the drawings below, the same or similar parts are indicated with the same or similar reference numerals. It should be noted that the drawings are schematic and that proportions of the dimensions and the like may be different from those in reality. Thus, specific dimensions and the like should be determined by taking the following explanations into consideration. Further, there may be some parts where relationships and proportions of dimensions vary among the drawings. In the cross-sectional views, although the constituent elements are indicated apart from one another for the sake of convenience in the explanations, the constituent elements in actual products are in contact with one another. An absorbent article 1 is a panty liner. The absorbent article 1 is used while being attached to a wearable article such as underwear.

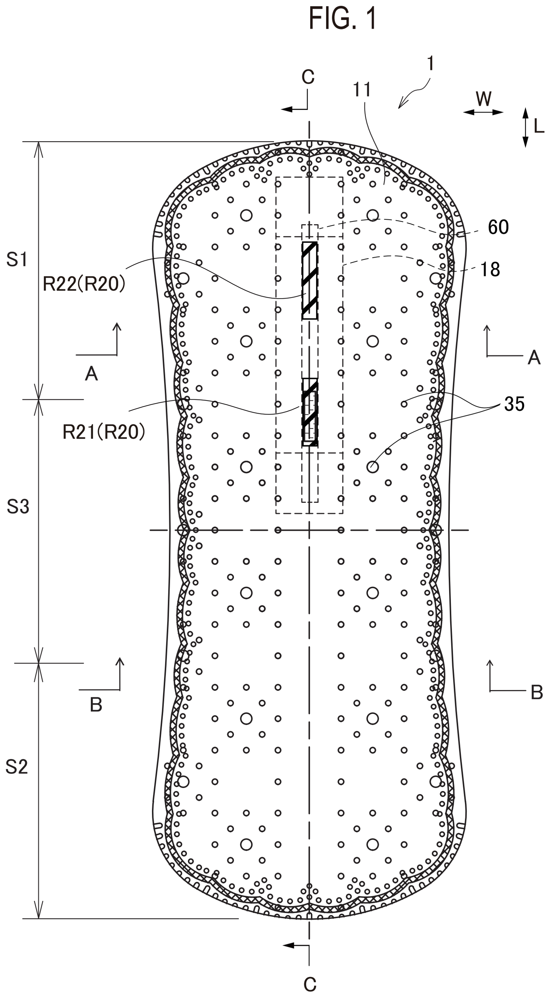

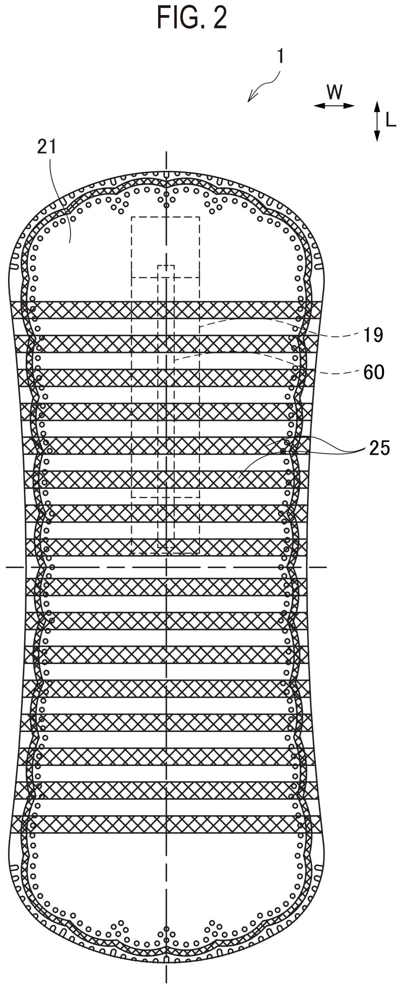

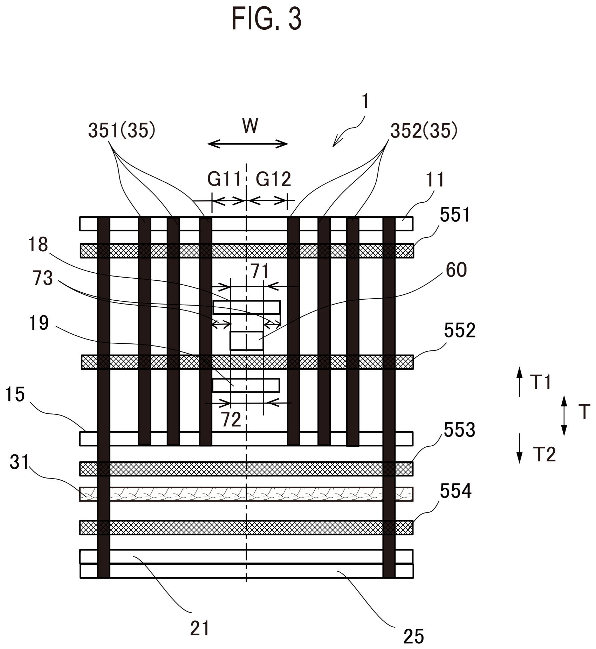

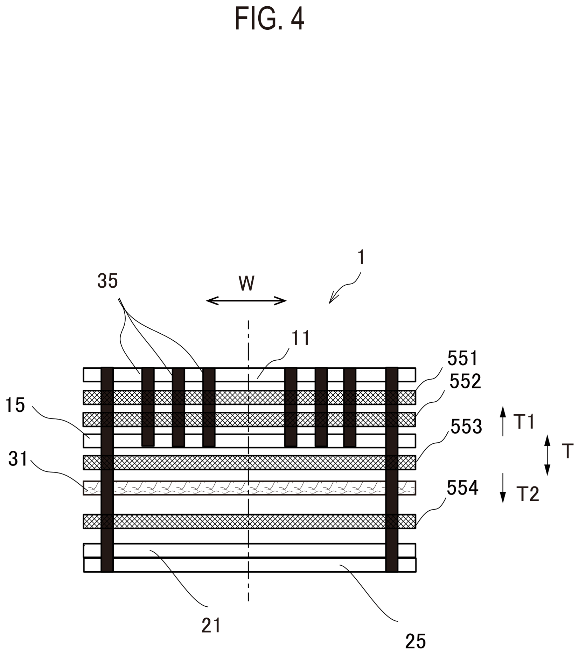

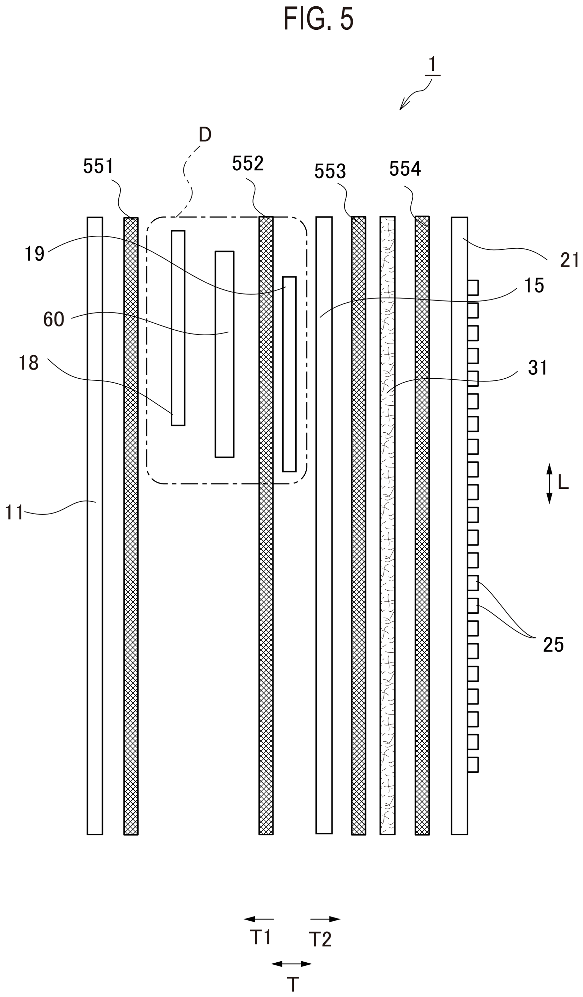

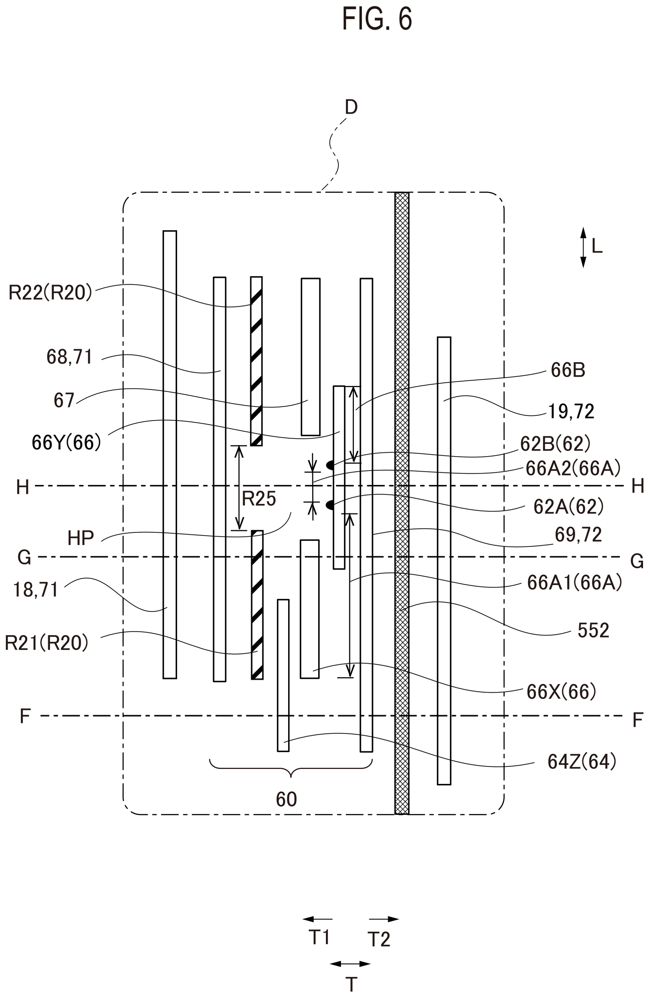

FIG. 1 is a plan view of the absorbent article 1 seen from a skin side T1. FIG. 2 is a plan view of the absorbent article 1 seen from a non-skin side T2. FIG. 3 is a cross-sectional view taken along line A-A indicated in FIG. 1. FIG. 4 is a cross-sectional view taken along line B-B indicated in FIG. 1. FIG. 5 is a cross-sectional view taken along line C-C indicated in FIG. 1. FIG. 6 is an enlarged view of a portion D indicated in FIG. 5. The absorbent article 1 may have a front-rear direction L, a width direction W, and a thickness direction T. The front-rear direction L is a direction extending from a front side (belly side) to a rear side (back side) of the wearer, or a direction extending from the rear side to the front side of the wearer. The width direction W is a direction orthogonal to the front-rear direction L. The thickness direction T is a direction orthogonal to the front-rear direction L and the width direction W. The thickness direction T extends to the skin side T1 and to the non-skin side T2. The skin side T1 corresponds to a side facing skin of the wearer during use. The non-skin side T2 corresponds to a side opposite from the skin side during use, that is, a side facing away from the skin of the wearer.

The absorbent article 1 has a front-side region S1, a rear-side region S2 and a crotch region S3. The crotch region S3 is a region that is positioned to be opposed to an excretion opening of the wearer, for example, a vaginal opening. The crotch region S3 is a region that is positioned in a crotch of the wearable article when the absorbent article 1 is attached to the wearable article and that lines between the legs of the wearer. The front-side region S1 is located frontward of the crotch region S3. A front end edge of the front-side region S1 defines the front end edge of the absorbent article 1. The rear-side region S2 is located rearward of the crotch region S3. A rear end edge of the rear-side region S2 defines the rear end edge of the absorbent article 1. The crotch region S3 may be a region located in a central one of three sections that are formed by trisecting the absorbent article 1 in the front-rear direction. In the absorbent article including wings, the crotch region S3 may be a region where the wings are disposed.

An outer portion in the present invention refers to a part that extends over a certain range in the width direction W, including an outer edge in the width direction W. The outer edge refers to an outer edge in the width direction W. Further, the outer edge of any constituent element extending over a certain range in the front-rear direction refers to an edge obtained by connecting points positioned on outer sides, in the width direction, of the constituent element, throughout the entire constituent element. An inner portion in the present invention refers to a part that extends over a certain range in the width direction W, including an interior edge in the width direction W. An inner edge refers to an interior edge in the width direction W. Further, the inner edge of any constituent element extending over a certain range in the front-rear direction refers to an edge obtained by connecting points positioned on inner sides, in the width direction, of the constituent element, throughout the entire constituent element. The front end and the rear end in the present invention each refer to a part that extends over a certain range in the front-rear direction L, including an edge in the front-rear direction L. The front end edge and the rear end edge each refer to an edge in the front-rear direction L. An outer end includes the front end and the rear end. An outer end edge includes the front end edge and the rear end edge. Further, an inner lateral side is a side that extends along the front-rear direction L, while including the inner edge. An outer lateral side is a side that extends along the front-rear direction L, while including the outer edge. In the present specification, the expression "along the front-rear direction L" denotes a direction oriented at an angle smaller than 45° with respect to the front-rear direction L. The expression "along the width direction W" denotes a direction oriented at an angle smaller than 45° with respect to the width direction W.

The absorbent article 1 may include at least an absorbent core 31, a topsheet 11, and a backsheet 21. The absorbent article 1 may be longitudinally elongated in shape. The absorbent core 31 includes an absorptive material that absorbs liquid. The absorptive material may be constituted of at least one of pulp and a superabsorbent polymer. The absorbent core 31 may be constituted of an air-laid pulp sheet. The absorbent core 31 may be sandwiched between core wrap sheets in the thickness direction.

The topsheet 11 configures a surface on the skin side of the absorbent article 1, and comes into contact with the skin of the wearer. The topsheet 11 may be constituted of, for example, a spunlace nonwoven fabric, an air-through nonwoven fabric, a spunbond nonwoven fabric, an SMS nonwoven fabric, and the like. Visible light transmittance of the topsheet 11 may be equal to or larger than 60%. The visible light transmittance of the topsheet 11 may be preferably equal to or larger than 70%, and more preferably equal to or larger than 80%. A basis weight of the topsheet 11 may be equal to or smaller than 90 g/m$^2$, equal to or smaller than 30 g/m$^2$, or equal to or smaller than 17 g/m$^2$. A thickness of the topsheet 11 may be equal to or smaller than 5 mm. A configuration of the topsheet 11 will be described in detail later.

The visible light transmittance can be measured by using a spectrophotometer (JASCO Corporation, V-570), for example. Specifically, the visible light transmittance can be measured by using a measurement target cut into 30 mm $\times$ 30 mm. In the following description, a wavelength range of visible rays is assumed to be 380 nm to 750 nm. Measurement conditions may be set as follows.

- Photometric mode: Abs
- Response: Medium
- Bandwidth: 2.0 nm
- Near-infrared: 8.0 nm
- Scanning speed: 1000 nm/min
- Start wavelength: 830 nm
- End wavelength: 350 nm
- Data acquisition interval: 1.0 nm

The backsheet 21 constitutes a surface on the non-skin side T2 of the absorbent article 1. In the absorbent article 1 attached to a wearable article, the backsheet 21 comes into contact with the wearable article. The backsheet 21 may be constituted of a liquid-impermeable film, for example, and may be a sheet having liquid impermeability and breathability. As the sheet having liquid impermeability and breathability, for example, it is possible to exemplify a film constituted of polyethylene resin and calcium carbonate, which is a sheet that has a surface on which a fine communication hole is formed, allows steam and air to permeate, and blocks water. On a surface on the non-skin side of the backsheet 21, a fastening portion 25 may be disposed for joining the absorbent article 1 with the wearable article (underwear and the like). The fastening portion 25 may be a part to which an adhesive agent such as a hot-melt type adhesive agent is applied, or a part where an engaging member such as a mechanical fastener is disposed. A plurality of the fastening portions 25 may be arranged to extend in the width direction W at intervals in the front-rear direction L.

The absorbent article 1 may include an auxiliary sheet 15 that is arranged on the skin side T1 relative to the absorbent core 31. The auxiliary sheet 15 enhances ability to draw in body fluid from the topsheet 11 into the absorbent core 31. The auxiliary sheet 15 may be constituted of an air-through nonwoven fabric. The auxiliary sheet may be arranged on the non-skin side T2 relative to a testing member 60, and on the skin side T1 relative to the absorbent core 31.

The absorbent article 1 may include a compressed region where a compressed part 35 obtained by compressing at least the topsheet 11 in the thickness direction T is formed, and an uncompressed region where the compressed part 35 is not formed. The compressed region is a region where the compressed part 35 is arranged in a plan view illustrated in FIG. 1, and the uncompressed region is a region where the compressed part is not arranged in a plan view. In the compressed part 35, at least the topsheet 11 may be compressed. Alternatively, the topsheet 11 and the auxiliary sheet 15 may be compressed, or the topsheet 11 and the absorbent core 31 may be compressed. The compressed part 35 in the present embodiment is configured by compressing the topsheet 11 and the auxiliary sheet 15. The compressed parts 35 may be arranged on both sides across the center in the width direction W of the absorbent article 1.

The absorbent article 1 includes the testing member 60 for testing a health condition of the wearer. The testing member 60 indicates the health condition of the wearer by color development (change in color) based on excreta. The testing member 60 may include a member that can hold or contain an indication drug, and may be constituted of any of materials such as paper, a nonwoven fabric, and a woven fabric, for example. Examples of excreta (a specimen) include blood (menstrual blood), urine, feces, sweat, vaginal discharge, and the like. Examples of items indicating the health condition include items related to a physical condition (pH, iron-deficiency anemia, a kidney function, myocardial infarction, inflammation/infectious disease, nutritional status assessment, and the like), items related to pregnancy (prediction of menstrual cycle, prediction of ovulation, and the like), and items related to mental health (depressive tendency, drugs, and the like). Examples of the detection target include waste products in urine (urine specific gravity), a white blood cell, a hydrogen ion (pH), protein, glucose, a ketone body, urobilinogen, bilirubin, occult blood in urine, nitrite, a steroid, peptide, an aromatic compound, a follicle-stimulating hormone (FSH), urea nitrogen (BUN), albumin (AIB), lipopolysaccharide (LPS), human chorionic gonadotropin (hCG), a luteinizing hormone (LH), U-ALB, C-reactive protein (CRP), myoglobin, CK-MB, troponin I, troponin T, hemoglobin, Strep A, an HBs antibody, an HIV antibody, a TP antibody, a rotavirus, an influenza virus, an adenovirus, DNA, O-157, cocaine, marijuana, morphine, ferritin, immunoglobulin A (IgA), and the like. The detection target is a chemical substance (inorganic substance, organic substance) that is determined in accordance with the item indicating the health condition, and causes color development by reacting with another chemical substance.

The testing member 60 in the present embodiment is a testing member using an immunochromatographic method for detecting a luteinizing hormone (LH) from vaginal discharge. The testing member 60 may detect the luteinizing hormone of 0.05 mIU/mL to 10.0 mIU/mL as positive. The absorbent article 1 is an article used in daily life unlike a conventional testing kit for a luteinizing hormone (LH) in urine, so that an ovulation day can be very easily predicted, and an effect of relatively reducing psychological resistance is exhibited. Further, to conventionally detect prediction of an ovulation day using urine, an operation of applying urine to a test piece is required and a relatively long time (for example, 10 minutes) is required in some cases. However, with the absorbent article 1, a detection reaction is caused following discharge of vaginal discharge only by attaching the absorbent article 1, so that the ovulation day can be more easily predicted. For example, with the absorbent article 1, instead of waiting for 10 minutes to check the testing result, the detection result can be easily checked at a timing of going to a toilet when 10 minutes have elapsed after the absorbent article 1 is attached. Unlike a conventional case of using urine, the absorbent article herein also has an advantage in that detection can be performed without concern about contamination of a hand. Thus, it is possible to provide an absorbent article that enables an ovulation day to be easily predicted.

Additionally, as a result of intensive study conducted by the applicant, a luteinizing hormone (LH) detected from vaginal discharge can be used to determine a predicted day in a range from five days before the ovulation day to the ovulation day itself. On the other hand, with a luteinizing hormone (LH) detected from urine, the predicted day is determined from two days including the ovulation day. More specifically, it is known that a testing member for detecting LH from urine typically detects a luteinizing hormone of 20.0 mIU/mL to 100.0 mIU/mL as positive. That is, a range of an amount of LH contained in urine is larger than a range of an amount of LH contained in vaginal discharge, so that sensitivity of the testing member for detecting LH from urine needs to be kept lower than that of the testing member for detecting LH in vaginal discharge for removing noise. The test member for detecting LH in urine may possibly detect peaks other than those in a fertile window, and it is difficult to design sensitivity of a testing appliance in the same numerical range as that of vaginal discharge. Thus, with the testing member for detecting LH in urine, it is difficult to perform prediction earlier than two days before the ovulation day using urine. On the other hand, with the testing member for detecting a luteinizing hormone (LH) from vaginal discharge, a predicted day five days before to three days before the ovulation day can be specified. Thus, with the testing member for detecting a luteinizing hormone (LH) from vaginal discharge, a predicted day five days before to three days before the ovulation day can be specified. A day five days before to three days before the ovulation day can be specified, so that conception activities can be planned and scheduled. As compared with a conventional form in which the ovulation day can be grasped on the ovulation day itself or a previous day, it is possible to enhance ability to carry out the conception activities while reducing a mental burden by reducing a burden of scheduling the conception activities on the ovulation day itself or preventing the conception activities from not being performed as it cannot be planned in advance. The ovulation day predicted based on a menstrual cycle is specified about two weeks before the ovulation day. Considering individual differences, prediction on a distant day such as two weeks before the ovulation day may lower a likelihood of accuracy thereof. There is also a disadvantage that the two weeks are too long in terms of planning. On the other hand, the testing member for detecting a luteinizing hormone (LH) from vaginal discharge can specify a predicted day five days before to three days before the ovulation day, predict the ovulation day on a day close to the ovulation day, improve accuracy in prediction of the ovulation day, and improve the ability to carry out the conception activities.

As illustrated in FIG. 6, the testing member 60 may include a movement portion 66 that allows an excreta component as a component contained in excreta to move to be guided to an indicator portion 62, and the indicator portion 62 that develops color based on the excreta component guided from the movement portion 66. The indicator portion 62 indicates a health condition of the wearer. The indicator portion 62 may indicate the health condition of the wearer by a color after color development, or may indicate the health condition of the wearer by presence/absence of color development. The wearer can check the health condition by determining a color after color development and/or presence/absence of color development by visual inspection. The indicator portion 62 may include an indication drug that develops color based on at least one of the excreta component and the labeled antibody. The indication drug may contain a chemical substance (inorganic substance, organic substance) that develops color by reacting with a detection target contained in excreta or a substance derived from the detection target (for example, a complex formed by an antigen-antibody reaction of the detection target and the antibody). The indication drug may also contain a chemical substance (inorganic substance, organic substance) that develops color by reacting with the labeled antibody (a chemical substance that causes an antigen-antibody reaction with the detection target) that has moved together with the excreta.

The testing member 60 may include a contact portion 64 with which the excreta is brought into contact. The contact portion 64 may be constituted of a pad (which may be referred to as a sample pad or a specimen pad), for example. The contact portion 64 may be configured to draw in excreta, and to guide an excreta component required for testing in the excreta to the movement portion 66. The indicator portion 62 may be constituted of a plurality of indicator portions having different detection targets. The indicator portion 62 may include, for example, a first indicator portion 62A and a second indicator portion 62B. The first indicator portion 62A is located to be closer to the contact portion 64 than the second indicator portion 62B.

In the movement portion 66, the excreta component may be moved by capillary action. The excreta component may contain the detection target. The movement portion 66 may include an indicator movement portion 66A in which the excreta component moves from the contact portion 64 to the indicator portion 62, and a terminal end movement portion 66B in which the excreta component moves in a direction away from the indicator portion 62. The indicator movement portion 66A may include a first indicator movement portion 66A1 from the contact portion 64 to the first indicator portion 62A, and a second indicator movement portion 66A2 in which the excreta component moves between a plurality of the indicator portions 62 (in the embodiment, from the first indicator portion 62A to the second indicator portion 62B). The terminal end movement portion 66B is a portion in which the excreta component and the like that have passed through the indicator portion 62 move.

To test the health condition, for example, an immunochromatographic method can be used. In the immunochromatographic method, the contact portion 64 to be brought into contact with excreta may be constituted of a sample pad or a specimen pad. The excreta component that has contacted the contact portion 64 moves in the indicator movement portion 66A. Specifically, the excreta component moves to the indicator portion 62 in the first indicator movement portion 66A1. The indicator portion 62 includes an antibody (labeled antibody) that reacts with the detection target (antigen). The labeled antibody may be included in the first indicator movement portion 66A1. In the first indicator movement portion 66A1, a part including the labeled antibody may be constituted of a conjugate pad, for example. In the first indicator movement portion 66A1, the excreta component, a complex formed by an antigen-antibody reaction with the detection target contained in the excreta component, and the labeled antibody may be moved by capillary action. The portion where these chemical substances move may be constituted of a membrane, for example. The movement portion 66 in the present embodiment includes a conjugate pad 66X and a membrane 66Y, and the labeled antibody is included in the conjugate pad 66X.

The indicator portion 62 is a region including an indication drug (capture antibody). The first indicator portion 62A may include the capture antibody that captures (couples) the complex. The first indicator portion 62A develops color when the complex is captured by the capture antibody. The first indicator portion 62A may be referred to as a test line. The complex, the labeled antibody, and the excreta component that have not been captured by the first indicator portion may move in the second indicator movement portion 66A2 to reach the second indicator portion 62B from the first indicator portion 62A.

The second indicator portion 62B may include a capture antibody different from that of the first indicator portion 62A. The capture antibody of the second indicator portion 62B may capture a complex different from the complex that has been captured by the first indicator portion 62A, or may capture the labeled antibody. The second indicator portion 62B develops color when the complex or the labeled antibody is captured by the capture antibody different from that of the first indicator portion 62A. The second indicator portion 62B that captures the labeled antibody may be referred to as a control line, which is an indicator for determining whether the testing has been correctly completed, and develops color when the testing is correctly completed. The excreta component that has passed through the indicator portion 62 (that is, the component that has not been captured by the capture antibody) moves in the terminal end movement portion 66B. The terminal end movement portion 66B may be constituted of the membrane 66Y. The testing member 60 may include an absorbent portion 67 that absorbs the excreta component that has passed through the indicator portion 62. The excreta component that has passed through the indicator portion 62 may be absorbed by the absorbent core 31 instead of the absorbent portion 67. In a case where the immunochromatographic method is used, the indicator portion 62 and the contact portion 64 are arranged at different positions.

The testing member 60 has a first direction as a movement direction of the excreta component from the movement portion 66 to the indicator portion 62, and a second direction orthogonal to the first direction. The first direction is a direction extending along the movement direction of the excreta component, which is a direction along the front-rear direction L of the absorbent article 1 in the present embodiment. That is, the first direction in the present embodiment is the front-rear direction L, and the second direction is the width direction W. However, in a modification, the first direction may be the width direction W, and the second direction may be the front-rear direction L. In the testing member 60, a length in the first direction may be longer than a length in the second direction.

The length of the testing member 60 along the width direction W may be equal to or larger than 1 mm and equal to or smaller than 10 mm. Although there are individual differences, a typical width of labia minora and the like is about 10 mm. By causing the length along the width direction W of the testing member to be shorter than the width of typical labia minora and the like, a sense of incongruity during wearing can be reduced. A length width along the width direction W of the testing member 60 may be preferably equal to or larger than 2 mm from a viewpoint of visibility of the wearer and processability. Preferably, the length along the width direction W of the testing member may be equal to or smaller than 3 mm. The length along the width direction W of the testing member is equal to or smaller than 3 mm, so that a liquid amount that needs to be retained for detection is relatively reduced. Thus, even when the detection target is vaginal discharge that is small in amount, high in viscosity, and low in LH amount, LH can be favorably detected.

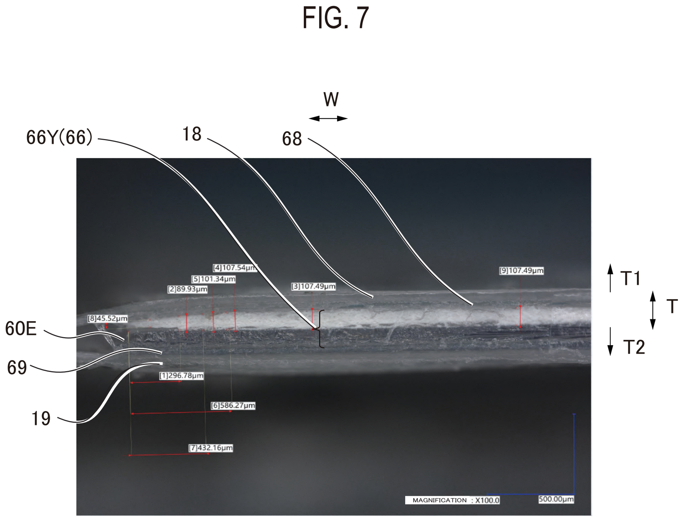

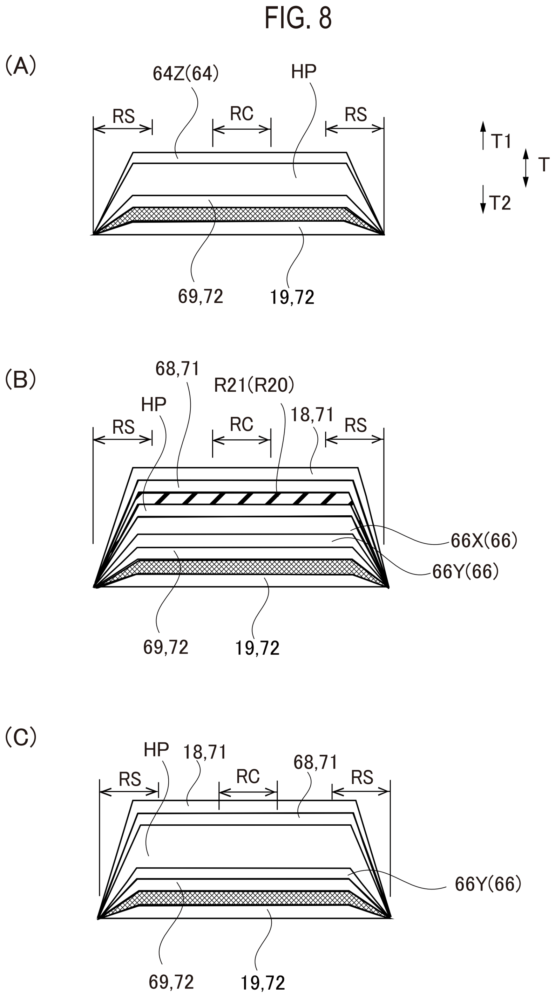

Subsequently, the following describes a preferred configuration of the testing member in detail. FIG. 7 is a cross-sectional photograph of a cross section taken along the width direction W of the testing member 60. FIG. 7 is a cross section of a region where the membrane 66Y is arranged. FIG. 8 is a diagram schematically illustrating a cross section taken along the width direction W of the testing member 60. FIG. 8(A) is a cross-sectional view taken along line F-F indicated in FIG. 6, FIG. 8(B) is a cross-sectional view taken along line G-G indicated in FIG. 6, and FIG. 8(C) is a cross-sectional view taken along line H-H. The cross-sectional views in FIG. 8 are schematic diagrams, and an actual surface on the skin side of the testing member 60 may have a curved shape that is smoothly inclined as illustrated in FIG. 7. More specifically, the surface on the skin side of the testing member 60 may have a shape that is inclined in a curved shape from an outer edge 60E of the testing member toward an inner side in the width direction and from the non-skin side T2 toward the skin side T1. An average thickness of a side region in a certain range from the outer edge 60E of the testing member toward the inner side in the width direction may be thinner than an average thickness of a center region. The testing member 60 in the present embodiment includes a sample pad 64Z as the contact portion 64, and the conjugate pad 66X and the membrane 66Y as the movement portion 66. The sample pad 64Z is arranged at one end in the front-rear direction of the testing member 60 (a rear end in the present embodiment). The conjugate pad 66X is arranged on the non-skin side T2 of the sample pad 64Z. The conjugate pad 66X is overlapped with another end in the front-rear direction L of the sample pad 64Z (a front end in the present embodiment) in the thickness direction, and is not overlapped with one end of the sample pad 64Z in the thickness direction T. Thus, excreta drawn into the sample pad 64Z is guided to the conjugate pad 66X after passing through the sample pad 64Z. The membrane 66Y is arranged on the non-skin side T2 of the conjugate pad 66X. The membrane 66Y is overlapped with another end of the conjugate pad 66X in the thickness direction T, and is not overlapped with one end of the conjugate pad 66X in the thickness direction. One end of the membrane 66Y and another end of the sample pad 64Z are separated from each other in the front-rear direction L. Thus, the excreta component moves through the sample pad 64Z, the conjugate pad 66X, and the membrane 66Y in order. The first indicator portion 62A and the second indicator portion 62B are disposed on the surface on the skin side T1 of the membrane 66Y. The first indicator portion 62A and the second indicator portion 62B are arranged at an interval in the front-rear direction L.

The testing member 60 in the present embodiment includes a testing skin side sheet 68, a testing non-skin side sheet 69, and the absorbent portion 67. The testing skin side sheet 68 is constituted of a liquid-impermeable film. The testing skin side sheet 68 constitutes the surface on the skin side T1 of the testing member 60, and is arranged to be closer to the skin side T1 than all of the sample pad 64Z, the conjugate pad 66X, the membrane 66Y, the absorbent portion 67, and the testing non-skin side sheet 69. The testing skin side sheet 68 does not cover the skin side T1 of a partial region of the sample pad 64Z (a region that is not covered by the conjugate pad 66X in the sample pad 64Z), and entirely covers the skin side T1 of the region covered by the conjugate pad 66X in the sample pad 64Z, the conjugate pad 66X, the membrane 66Y, and the absorbent portion 67. The testing non-skin side sheet 69 constitutes the surface on the non-skin side T2 of the testing member 60, and is arranged to be closer to the non-skin side than all of the sample pad 64Z, the conjugate pad 66X, the membrane 66Y, the absorbent portion 67, and the testing skin side sheet 68. The testing non-skin side sheet 69 entirely covers the non-skin side T2 of the sample pad 64Z, the conjugate pad 66X, the membrane 66Y, the absorbent portion 67, and the testing skin side sheet 68. The absorbent portion 67 is overlapped with another end in the front-rear direction L of the membrane 66Y in the thickness direction T, and is not overlapped with one end of the membrane 66Y in the thickness direction T. The absorbent portion 67 is arranged on the skin side T1 relative to the membrane 66Y.

The labeled antibody of the testing member 60 may include a colored antibody bound to a molecule contributing to coloration. The labeled antibody may be constituted of only the colored antibody, or may include an antibody other than the colored antibody. The colored antibody is colored in advance before color development. The colored antibody may be colored in a color different from that of a member in which the colored antibody is arranged (for example, the conjugate pad). Examples of the coloration of the colored antibody include blue, red, yellow, and white, for example. In a plan view of the absorbent article 1, a color difference between the part in which the colored antibody is arranged and surroundings of the colored antibody (in the present embodiment, a part in which the colored antibody is not arranged in the conjugate pad) may be equal to or larger than 3.0. The part in which the colored antibody is arranged in the present embodiment is the conjugate pad 66X. A complex formed by an antigen-antibody reaction caused by the colored antibody has color that is colored based on the colored antibody.

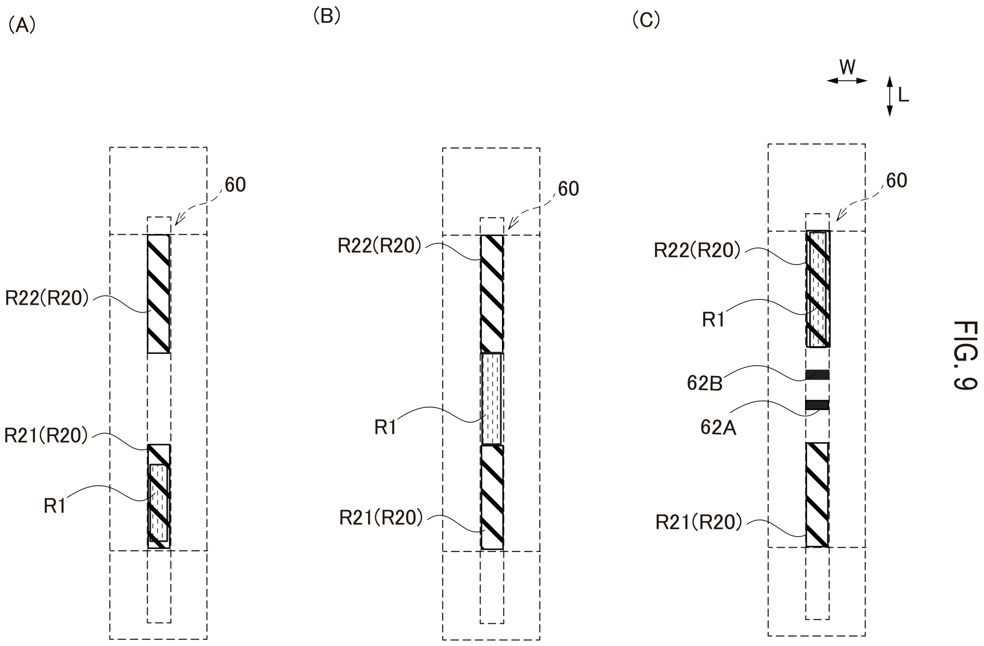

FIG. 9 illustrates an example of a color developing aspect of the indicator portion 62 in a plan view of the testing member 60 viewed from the skin side T1. In FIG. 9, a region colored by the colored antibody is indicated by R1, and a colored region described later is indicated by R20. FIG. 9(A) illustrates the testing member 60 before both of the first indicator portion 62A and the second indicator portion 62B show a color developing reaction (hereinafter, referred to as "before color development"), FIG. 9(B) illustrates a state during testing, and FIG. 9(C) illustrates the absorbent article 1 after both of the first indicator portion 62A and the second indicator portion 62B show a color developing reaction (hereinafter, referred to as "after color development"). FIG. 9(C) illustrates a color developing aspect in a case where a detection target is detected (in a case where the test line develops color). In the state before color development illustrated in FIG. 9(A), the first indicator portion 62A and the second indicator portion 62B are integrated with the surroundings. A state in which the first indicator portion 62A and the second indicator portion 62B are integrated with the surroundings is a state in which a color difference between the first indicator portion 62A and the surrounding membrane 66Y is smaller than 3.0, and a state in which the color difference between the second indicator portion 62B and the surrounding membrane is smaller than 3.0. On the other hand, a state in which the first indicator portion 62A and the second indicator portion 62B can be recognized to be distinguished from the surroundings (not integrated with the surroundings) is a state in which the color difference between the first indicator portion 62A and the surrounding membrane 66Y is equal to or larger than 3.0, and a state in which the color difference between the second indicator portion 62B and the surrounding membrane 66Y is equal to or larger than 3.0. In the state before color development, it is difficult for the wearer to recognize the first indicator portion 62A and the second indicator portion 62B.

As illustrated in FIG. 9(B), during testing, the complex and the colored antibody move from the conjugate pad 66X, and a skin surface of the membrane 66Y is colored by the colored antibody and the complex colored by the colored antibody. The wearer can see the colored antibody and the colored complex, and grasp the fact that the testing is in progress. As the testing further progresses, the complex and the colored antibody move through the second indicator movement portion 66A2, and move to the terminal end movement portion 66B. FIG. 9(C) is a state after color development. The first indicator portion 62A develops color by the complex, and the second indicator portion 62B develops color by the labeled antibody (including the colored antibody). The complex and the labeled antibody that are not captured by the first indicator portion 62A and the second indicator portion 62B move to the absorbent portion 67, and are adsorbed by the absorbent portion 67. In this manner, when the colored antibody is provided, the wearer can grasp a testing process. The indicator portion develops color by capturing at least one of the complex and the labeled antibody. When the complex is colored, the color developed by capturing the complex tends to be noticeable. The color developed by capturing the colored antibody also tends to be noticeable. Accordingly, the indicator portion after color development can be made more noticeable, and the wearer is enabled to easily grasp a testing result.

However, when seeing the testing member 60 before use, some wearers may erroneously perceive a state before the color developing reaction as a state after the color developing reaction by seeing the colored antibody. The absorbent article 1 according to the present embodiment is configured to prevent erroneous recognition of the wearer. More specifically, the absorbent article 1 includes a colored region R20 that covers the skin side T1 of the colored antibody disposed at the movement portion 66. The colored region R20 may cover the skin side T1 of the colored antibody before use of the testing member 60, that is, in a state before the colored antibody moves, and does not necessarily cover the skin side T1 of the colored antibody that has moved in the testing process. Before use of the absorbent article 1, as the skin side T1 of the colored antibody disposed at the movement portion 66 is covered by the colored region R20, the colored antibody is arranged on a back surface side of the colored region R20 when the wearer sees the absorbent article 1 before use from the skin side T1, and it is difficult to directly see the colored antibody. The wearer hardly sees the colored antibody before use, so that it is possible to prevent erroneous recognition such that the testing member 60 has been already reacted before use of the absorbent article 1.

The colored region R20 is configured to be visually recognizable from the skin side T1 of the absorbent article 1 before use of the absorbent article 1. Due to this, the wearer can recognize the absorbent article 1 including the testing member 60 by seeing the colored region R20, and can recognize presence of the testing member 60 and necessity to grasp a testing result. The absorbent article 1 is used not only in a case of performing testing but also in daily life, and both of the absorbent article 1 including the testing member 60 and the absorbent article not including the testing member 60 may be appropriately used. A user can recognize the absorbent article 1 including the testing member 60 by seeing the colored region R20. Even when the absorbent article including the testing member 60 is attached for performing testing, the user may forget that the testing is being performed during wearing. Even in such a case, the user can once again recognize the presence of the testing member 60 when seeing the absorbent article 1 such as a time when the absorbent article 1 is replaced, and can appropriately grasp a testing result.

The colored region R20 may be disposed in a member located on the skin side T1 of the colored antibody among members constituting the absorbent article 1. The colored region R20 may be disposed in the testing member 60 itself, or may be disposed in a member located on the skin side T1 relative to the testing member 60 (for example, a topsheet, a skin side cover sheet described later, and the like). The colored region R20 may be disposed in one layer of sheet, or may be disposed in a plurality of layers so that colored regions are overlapped with each other in the thickness direction. The colored region R20 may be a portion obtained by coloring a sheet itself by a pigment and the like, or a portion obtained by partially coloring a sheet by printing and the like. The colored region R20 may cover at least part of the colored antibody before color development, and may preferably cover the entire colored antibody (the entire region R1 colored by the colored antibody). In a form of including both of the colored antibody and the antibody that is not coupled with a molecule contributing to coloration, the colored region R20 may cover at least the colored antibody, and does not necessarily cover the antibody that is not coupled with the molecule contributing to coloration. The colored region R20 in the present embodiment is disposed on a non-skin surface of the testing skin side sheet 68 constituting the testing member. The testing skin side sheet 68 is constituted of a transparent resin film. A portion colored by ink and the like in the transparent resin film constitutes the colored region R20, and a portion that is not colored constitutes a high transmittance region R25. Before color development, the colored region R20 covers the region R1 colored by the colored antibody in the conjugate pad 66X.

The testing member 60 may be visually recognizable from the skin side T1 of the absorbent article 1 before use of the absorbent article 1. Before use of the absorbent article 1, the indicator portion 62 is in a state before color development. Thus, visibility of the testing member 60 before use of the absorbent article can be replaced with visibility of the testing member 60 before color development of the indicator portion 62. The state in which the testing member 60 is visually recognizable before use of the absorbent article 1 may be a state in which at least part of the testing member 60 before color development is visually recognizable. It is sufficient that the user can grasp the presence of the testing member 60. The state includes not only a configuration in which the testing member 60 is directly visually recognizable but also a state in which a surface on the skin side of the absorbent article 1 bulges due to the testing member 60 and the presence of the testing member is recognizable due to the bulging portion. The testing member 60 is visually recognizable from the skin side T1 of the absorbent article 1 before use of the absorbent article 1, so that a position of the testing member 60 in the absorbent article 1 can be grasped at the time of attachment. When the wearer attaches the absorbent article 1 with awareness of the presence of the testing member 60, the testing member 60 can be arranged at an appropriate position.

The indicator portion 62 after color development may be visually recognizable from the skin side T1 of the absorbent article 1. The indicator portion 62 after color development is visually recognizable from the skin side T1 of the absorbent article 1, so that the wearer can easily grasp a testing result by seeing the absorbent article 1 from the skin side T1 of the absorbent article 1 without pulling off the absorbent article 1 from the wearable article or taking off the absorbent article 1 from the wearable article. The indicator portion 62 may be visually recognizable from the skin side T1 of the absorbent article 1 at least after color development, and may be visually unrecognizable from the skin side T1 of the absorbent article 1 before color development, or may be visually recognizable from the skin side T1 of the absorbent article 1 before color development.

The state of being visually recognizable from the skin side of the absorbent article 1 may be a state in which visible light transmittance is equal to or larger than 60% when a member positioned on the skin side relative to an object the visibility of which is evaluated (for example, the testing member in a case of evaluating visibility of the testing member 60) is overlapped. Alternatively, the state of being visually recognizable from the skin side of the absorbent article 1 means that, when a subject having favorable eyesight (1.0 or more) in both eyes can visually recognize the object when seeing the object from the skin side T1 of the absorbent article 1 at a distance of about 30 to 50 cm in a room brightly illuminated (rough measure: 500 to 750 lx (lux)) with daylight-white light (rough measure of color temperature: 4600 to 5400 K (kelvin)).

The testing member 60 may be arranged on the non-skin side T2 relative to the topsheet 11, and on the skin side T1 relative to the backsheet 21. The testing member 60 is seen from the skin side T1 of the absorbent article 1 via the topsheet 11. Visible light transmittance of the topsheet 11 may be equal to or larger than 60%. Due to this, the wearer can see the color of the indicator portion after color development more clearly via the topsheet 11, so that the wearer can grasp whether the indicator portion 62 has developed color more easily. A color difference between the topsheet 11 and the indicator portion 62 after color development may be equal to or larger than 0.6, and preferably equal to or larger than 3.0. According to this configuration, it becomes difficult for the color of the indicator portion 62 after color development to be similar to the color of the topsheet 11, thereby allowing the indicator portion 62 after color development to be easily visually recognized. The user is enabled to easily grasp whether the indicator portion has developed color without taking out the testing member 60 from the absorbent article, and the health condition of the user can be easily checked with high accuracy.

In a state before color development of the indicator portion 62, the testing member 60 may be visually recognizable from the skin side T1 of the absorbent article 1 and may be visually unrecognizable from the non-skin side T2 of the absorbent article 1. The testing member 60 can be visually recognized from the skin side T1 of the absorbent article 1 in the state before color development of the indicator portion 62, so that the position of the testing member 60 in the absorbent article 1 can be grasped at the time of attachment, and the testing member 60 can be arranged at an appropriate position. The testing member 60 cannot be visually recognized from the non-skin side T2 of the absorbent article 1 in the state before color development of the indicator portion 62, so that the skin surface and the non-skin surface of the absorbent article 1 can be intuitively grasped depending on whether the testing member 60 is visually recognizable, and operability at the time of attachment can be improved. The colored antibody may be visually unrecognizable from the non-skin side T2 of the absorbent article 1 before use of the absorbent article 1. Accordingly, also when the absorbent article 1 is seen from the non-skin side T2, it is possible to prevent erroneous recognition before use of the absorbent article 1.

The state of being visually unrecognizable from the non-skin side T2 of the absorbent article 1 may be a state in which visible light transmittance is equal to or smaller than 20% when a member positioned on the non-skin side T2 relative to the object the visibility of which is evaluated (for example, the testing member in a case of evaluating visibility of the testing member 60) is overlapped. Alternatively, the state of being visually unrecognizable from the non-skin side of the absorbent article 1 means that, when the subject having favorable eyesight (1.0 or more) in both eyes cannot visually recognize the object when seeing the object from the non-skin side T2 of the absorbent article 1 at a distance of about 30 to 50 cm in a room brightly illuminated (rough measure: 500 to 750 lx (lux)) with daylight-white light (rough measure of color temperature: 4600 to 5400 K (kelvin)).

The testing member 60 may be arranged on the skin side T1 relative to the absorbent core 31. The testing member 60 is located on the skin side T1 relative to the absorbent core 31, and the skin side T1 is not covered by the absorbent core 31. Thus, visibility of the testing member 60 can be easily secured when being seen from the skin side T1 of the absorbent article 1. At the time of attaching the absorbent article 1, the wearer can attach the absorbent article 1 with awareness of the presence of the testing member 60, and the testing member 60 can be arranged at an appropriate position. After testing of the testing member 60, the wearer can easily grasp a testing result.

The topsheet 11 and the backsheet 21 are constituted of sheets including fibers, and an average inter-fiber distance of the backsheet 21 may be smaller than an inter-fiber distance of the topsheet 11. With this configuration, before color development of the indicator portion 62 and after color development of the indicator portion 62, the testing member 60 is easily visually recognized from the skin side T1 of the absorbent article 1, and the testing member 60 is difficult to be visually recognized from the non-skin side T2 of the absorbent article 1. The visibility of the testing member 60 from the skin side T1 of the absorbent article 1 can be easily secured, and operability at the time of attachment can be improved by improving a discrimination property between the skin surface and the non-skin surface of the absorbent article 1. The average inter-fiber distance can be evaluated, for example, by measuring an average inter-fiber distance by a method using a microscope on a surface as a measurement target (a surface on the skin side of the topsheet 11), and measuring the average inter-fiber distance using a method using a microscope on a surface on the non-skin side of the backsheet 21. In the method of using the microscope, a 3D image connection function of the microscope (VHX-2000 manufactured by KEYENCE CORPORATION, lens VH-Z20W with an aperture fully opened) is used to obtain a magnified image in which focus is identical from a skin side surface of a sample to a depth of 100 $\mu$m, and an outer side of a fiber in focus is extracted based on the magnified image. A surface formed therein is assumed to be a fiber space. A diameter of a maximum inscribed circle of the fiber space is assumed to be a fiber space distance, and an average value of 100 points in the fiber space is assumed to be an average inter-fiber distance. A fiber density of the backsheet 21 may be higher than a fiber density of the topsheet 11. Fibers of the backsheet 21 are dense and fibers of the topsheet 11 are sparse, so that visibility via the backsheet 21 is low and visibility via the topsheet 11 is high. Accordingly, the testing member 60 can be easily seen from the skin side T1 of the absorbent article 1, and the testing member 60 is difficult to be seen from the non-skin side T2 of the absorbent article 1.

As illustrated in FIG. 8, of regions obtained by equally dividing the testing member 60 into five regions in the second direction (in the present embodiment, the width direction W), the testing member 60 includes a center region RC located at the center in the second direction of the testing member 60, and side regions RS located at both ends in the second direction of the testing member 60. The center region RC straddles the center in the second direction of the testing member 60. The side region RS extends from an outer end edge in the second direction of the testing member 60 toward an inner side in the second direction (in a form in which the second direction is the width direction as in the present embodiment, from the outer edge 60E in the width direction of the testing member toward the inner side in the width direction). The indicator portion 62 may be arranged at least in the center region RC. The indicator portion 62 may extend toward an outer side in the second direction with respect to the center region RC, may be arranged between the center region RC and the side region RS, or may be continuously arranged from the center region RC to the side region RS.

An average thickness of the center region RC of the testing member 60 may be thicker than an average thickness of the side region RS of the testing member 60. The average thickness of the center region RC is thicker than that of the side region RS, and it is possible to secure a large capacity of a space in which the excreta component moves. Thus, the excreta component can easily move along the first direction in the center region RC, and a color developing reaction is easily caused at the indicator portion 62 in the center region RC. When the color developing reaction is caused in the center region RC located at the center in the second direction of the testing member 60, the wearer can easily see the color developing reaction, and visibility of a testing result can be improved. Additionally, regions for absorbing excreta such as the absorbent core and the topsheet 11 are arranged around an end edge in the second direction of the testing member, so that visibility may be lowered due to excreta absorbed by the surrounding regions. On the other hand, the center region RC is located at a position distant from excreta absorbed by the surrounding regions, so that visibility is hardly lowered due to the excreta. Thus, when the color developing reaction is caused at the indicator portion 62 in the center region RC, the wearer can easily grasp a testing result. The average thickness may be an average value of thicknesses measured at ten points in each region using a cross-sectional photograph (for example, refer to FIG. 7) of a cross section along the second direction. Each of the thickness of the center region RC and the thickness of the side region RS is a distance along the thickness direction between a skin side surface of the testing member 60 and a non-skin side surface of the testing member 60 in each region. The thickness of the indicator portion is a distance along the thickness direction between a skin side surface of the indicator portion and a non-skin side surface of the indicator portion. The thickness of the topsheet 11 is a distance along the thickness direction between a skin side surface of the topsheet 11 and a surface of the topsheet 11. The side regions RS are respectively arranged at both ends in the second direction of the testing member. The average thickness of at least one of the side regions may be thinner than the average thickness of the center region RC. Preferably, the average thicknesses of both side regions may be thinner than the average thickness of the center region RC.

As illustrated in FIG. 6, for example, the testing member 60 is constituted of a plurality of members having respective functions such as the movement portion 66 and the indicator portion 62. The topsheet 11 may cover at least part of the testing member, or may cover the entire testing member 60. In a configuration in which the average thickness of the side region RS is thinner than the average thickness of the center region RC and the topsheet 11 covers the entire testing member 60, an end edge of the testing member 60 is hardly hooked even when external force is applied to the testing member 60 due to movement of the wearer and the like during wearing. Accordingly, it is possible to prevent curling up at the end edge of the testing member 60 and misalignment of members constituting the testing member, and testing accuracy can be maintained. By preventing curling up and twist at the end edge of the testing member, a thickness and density of each constituent element can be prevented from being locally changed. When the thickness and the density are locally changed, movement of the excreta component may stagnate or a color developing reaction may be hardly caused at the changed portion. However, the thickness and the density of each constituent element can be prevented from being locally changed, and testing accuracy can be prevented from being lowered.

A density of the center region RC is lower than a density of the side region RS. The density can be calculated by cutting out each region with the same plane dimensions in a range including a cross section with which the average thickness is measured, and using a weight of each region and the average thickness described above. The center region RC is sparser than the side region RS within the constituent elements and between the constituent elements, and the side region RS is denser than the center region RC within the constituent elements and between the constituent elements. The excreta component that moves in the testing member 60 easily moves in the center region RC that is relatively sparse, and hardly moves in the side region RS that is relatively dense. Thus, the excreta component can easily move in the first direction in the center region RC, and a color developing reaction is easily caused at the indicator portion in the center region RC. When the color developing reaction is caused at the center in the second direction of the testing member, the wearer can easily see the color developing reaction, and visibility of a testing result can be improved.

At the cross section along the second direction, the average thickness of the indicator portion 62 in the center region RC may be thicker than the average thickness of the indicator portion 62 in the side region RS. A color developing reaction is easily caused at the indicator portion 62 in the center region RC. A color developing reaction is easily caused at the center in the second direction of the testing member 60, so that the wearer can easily see the color developing reaction, and visibility of a testing result can be improved. In a form in which the indicator portion is constituted of the indication drug arranged on the membrane as in the present embodiment, an average thickness of the membrane in the center region RC may be thicker than an average thickness of the membrane in the side region RS.

An average thickness of the topsheet 11 in a region covering the side region RS may be thicker than an average thickness of the topsheet 11 in a region covering the center region RC. The average thickness of the topsheet 11 covering the center region RC is thin, so that body fluid can be quickly guided from the topsheet 11 side to the testing member 60 side without continuously holding the body fluid in the topsheet 11. Thus, a color developing reaction is easily caused at the indicator portion 62 in the center region RC. When the color developing reaction is caused at the center in the second direction of the testing member 60, the wearer can easily see the color developing reaction, and visibility of a testing result can be improved. The side regions RS are respectively arranged at both ends in the second direction of the testing member. The average thickness of the topsheet 11 covering at least one of the side regions may be thicker than the average thickness of the topsheet 11 covering the center region RC, but preferably, the average thickness of both sides of the topsheet 11 covering both side regions may be thicker than the average thickness of the topsheet 11 covering the center region RC.

With the configuration in which the average thickness of the topsheet 11 in the region covering the side region RS is thicker than the average thickness of the topsheet 11 in the region covering the center region RC and the entire testing member 60 is covered by the topsheet 11, the thickness of the topsheet 11 covering the side region RS is relatively thick, so that the topsheet 11 can absorb external force applied to the end edge of the testing member, and it is possible to prevent curling up at the end edge of the testing member and misalignment of members constituting the testing member.

A density of the topsheet 11 in the region covering the center region RC may be higher than a density of the topsheet 11 in the region covering the side region RS. The density of the topsheet 11 covering the center region RC is relatively high and the density of the topsheet 11 covering the side region RS is relatively low, so that excreta can be guided from the side region RS to the center region RC side in the topsheet 11, and the excreta component can be guided to the center in the second direction to promote a color developing reaction at the center in the second direction of the testing member.

To further promote the color developing reaction at the center in the second direction of the testing member 60, the indicator portion 62 at the center in the second direction may be configured to be noticeable. For example, an area ratio of the indicator portion 62 in the center region RC may be configured to be higher than an area ratio of the indicator portion 62 in the side region RS. Alternatively, a basis weight of the indication drug constituting the indicator portion 62 may be varied. The basis weight of the indication drug in the center region RC may be higher than the basis weight of the indication drug in the side region RS.

The first direction may be the front-rear direction L, and the second direction may be the width direction W. The absorbent article is held between legs during wearing, and is likely to receive a force directed from an outer side toward an inner side in the width direction. Thus, the outer edge as the end edge in the width direction of the testing member is likely to receive external force more easily than the center in the width direction of the testing member. In a form in which the average thickness of the side region is thinner than the average thickness of the center region, the relatively thin side region hardly receives the external force, so that an outer portion of the testing member can be prevented from being deformed, and a color developing reaction based on the excreta component can be caused without inhibiting movement of the excreta component. In a form in which the average thickness of the topsheet in the region covering the side region is thicker than the average thickness of the topsheet in the region covering the center region, the relatively thick topsheet can absorb an external force applied to the side region, the outer portion of the testing member can be prevented from being deformed, and a color developing reaction based on the excreta component can be caused without inhibiting movement of the excreta component. In a form in which the density of the side region located at the outer portion of the testing member is high, the outer portion of the testing member can be prevented from being deformed, and a color developing reaction based on the excreta component can be caused without inhibiting movement of the excreta component.

The testing member 60 may be arranged offset toward one side in the front-rear direction with respect to the center of the front-rear direction L of the absorbent article 1, and the contact portion 64 may be arranged at an end on the other side of the front-rear direction of the testing member 60. For example, the testing member 60 may be offset toward the front side with respect to the center in the front-rear direction L of the absorbent article 1, and the contact portion 64 may be arranged at the rear end of the testing member 60. Alternatively, the testing member 60 may be offset toward the rear side with respect to the center in the front-rear direction L of the absorbent article 1, and the contact portion 64 may be arranged at the front end of the testing member 60. The contact portion 64 is arranged at the end on the other side in the front-rear direction of the testing member, located on the center side in the front-rear direction of the absorbent article, and tends to be arranged in the vicinity of the excretion opening. Accordingly, excreta can be smoothly drawn in by the contact portion. Preferably, the testing member 60 may be offset toward the front side with respect to the center in the front-rear direction L of the absorbent article 1. With this configuration, the testing member 60 is likely to be in sight of the wearer, and the wearer can easily grasp presence of the testing member 60 and can easily see a testing result. The end on the other side of the contact portion 64 is not covered by the skin side sheet described later, and may be covered by the non-skin side sheet. The end on the other side of the contact portion is covered by the liquid-impermeable non-skin side sheet to prevent contact with excreta from the non-skin side, and is not covered by the skin side sheet, so that excreta from the skin side can be easily brought into contact therewith.

The testing member 60 may be arranged at a center portion in the width direction W of the absorbent article 1. At least part of the testing member 60 may be arranged at the center portion in the width direction W of the absorbent article 1. With this configuration, the contact portion 64 is easily arranged to be opposed to the excretion opening. By arranging the testing member 60 to be opposed to the excretion opening, excreta can be easily guided to the testing member 60, and the wearer can easily grasp a testing result after testing of the testing member. The center portion in the width direction W of the absorbent article 1 may be a range including the center in the width direction W of the absorbent article 1, and extending from the center toward the outer side in the width direction by 10 mm. Preferably, it may be arranged while straddling the center in the width direction W of the absorbent article 1. The contact portion 64 of the testing member 60 may be arranged in the crotch region S3.

In a plan view of the absorbent article, the testing member 60 may be arranged to be inclined with respect to the front-rear direction L of the absorbent article 1. A form in which the testing member 60 is arranged to be inclined with respect to the front-rear direction L may be a form in which the outer edge in the width direction W of the testing member 60 is not parallel with but inclined with respect to the front-rear direction L, or a form in which a testing center line connecting the center in the width direction W at the front end of the testing member 60 and the center in the width direction W at the rear end of the testing member 60 is not parallel with but inclined with respect to the front-rear direction L. When the testing member 60 is inclined with respect to the front-rear direction L of the absorbent article 1, the testing member 60 is likely to be more noticeable than outer edges of the other constituent elements (for example, the topsheet and the absorbent core) of the absorbent article 1, so that the user can easily grasp the presence of the testing member 60. Preferably, the length in the front-rear direction L of the testing member 60 may be longer than the length in the width direction W of the testing member 60. The wearer can easily grasp a state in which the testing member 60 is inclined.

An angle formed by the testing center line of the testing member 60 and the front-rear direction L may be equal to or smaller than 20 degrees, and preferably equal to or larger than 3 degrees and equal to or smaller than 10 degrees.

The compressed part 35 may include a first compressed part 351 and a second compressed part 352 arranged on opposite sides across the testing member 60 in the width direction W. The first compressed part 351 and the second compressed part 352 are arranged at an interval in the width direction W, and the testing member 60 may be arranged between the first compressed part 351 and the second compressed part 352. As illustrated in FIG. 3, a distance G11 in the width direction between the first compressed part 351 and the testing member 60 may be different from a distance G12 in the width direction W between the second compressed part 352 and the testing member 60. When comparing the distance G11 with the distance G12, the comparison is made on the same imaginary line extending in parallel with the width direction W. In a form in which a plurality of first compressed parts 351 and a plurality of second compressed parts 352 are provided, the comparison is made with the compressed parts having shapes line-symmetrical with respect to a center line passing through the center in the width direction of the absorbent article 1 and extending in the front-rear direction L. The first compressed part 351 and the second compressed part 352 may extend in the front-rear direction L. When the first compressed part 351 and the second compressed part 352 extend in the front-rear direction L, the testing member can be easily made relatively noticeable with respect to the first compressed part and the second compressed part. The configuration in which the compressed part 35 extends in the front-rear direction L includes not only a line shape extending in the front-rear direction but also arrangement in which an assembly of a plurality of compressed parts arranged at intervals extends in the front-rear direction.

On the outer portion of the testing member 60, a projecting part projecting toward the outer side in the width direction W and a recessed part recessed toward the inner side in the width direction W with respect to the projecting part may be formed. The projecting part and the recessed part may be alternately formed side by side in the front-rear direction L. With this configuration, the outer portion of the testing member 60 is likely to be noticeable, and the user can easily grasp the presence of the testing member 60. A length in the width direction of the projecting part and a length in the width direction of the recessed part may be equal to or larger than 20 μm and equal to or smaller than 60 μm, and a length in the front-rear direction of the projecting part and a length in the front-rear direction of the recessed part may be equal to or larger than 50 μm and equal to or smaller than 400 μm.

At a cross section along the front-rear direction L, the skin side surface of the testing member 60 may be inclined toward the non-skin side T2 from the center in the front-rear direction L of the testing member 60 toward the outer end of the testing member 60. The skin side surface is inclined toward the outer side in the front-rear direction L of the testing member 60 and toward the non-skin side T2, so that, when body fluid reaches the skin side surface of the testing member 60, the body fluid can be guided to the outer side in the front-rear direction L of the testing member 60 while being guided to the non-skin side T2. Thus, the body fluid hardly remains on the testing member 60, and it is possible to prevent the body fluid from reaching the inside of the testing member 60 via an unintended path. The body fluid hardly remains on the testing member 60, so that it is possible to prevent visibility from being lowered due to remaining body fluid.

As illustrated in FIG. 7 and FIG. 8, at the cross section along the width direction W, the skin side surface of the testing member 60 may be inclined toward the non-skin side T2 from the center in the width direction W of the testing member 60 toward the outer portion of the testing member 60. The skin side surface is inclined toward the outer side in the width direction W of the testing member 60 and toward the non-skin side T2, so that, when body fluid reaches the skin side surface of the testing member 60, the body fluid can be guided to the outer side in the width direction W of the testing member 60 while being guided to the non-skin side T2. Thus, the body fluid hardly remains on the testing member 60, and it is possible to prevent the body fluid from reaching the inside of the testing member 60 via an unintended path. The body fluid hardly remains on the testing member 60, so that it is possible to prevent visibility from being lowered due to a remaining body fluid. In determining whether the skin side surface of the testing member is inclined toward the non-skin side, whether there is an inclined portion may be checked by visual recognition based on a photograph of a cross section along the front-rear direction of the testing member or a cross section along the width direction. Alternatively, the testing member may be equally divided into five portions in the front-rear direction or the width direction, and a position of a skin surface of a portion located at the center may be compared with a position of a skin surface located at an end to determine whether the skin side surface of the testing member is inclined toward the non-skin side. The configuration in which the skin side surface of the testing member is inclined toward the non-skin side may be a configuration in which an end of the testing member is pressed more than the center to have a higher density, or a configuration in which the number of materials or a basis weight of the material at the end of the testing member is smaller than that at the center.

As illustrated in FIG. 8, the skin side sheet may be inclined toward the non-skin side from the center in the width direction W of the skin side sheet toward the outer edge in the width direction W of the skin side sheet. Excreta discharged onto the skin side sheet are guided to the non-skin side from the center in the width direction toward a lateral side in the width direction along the skin side sheet. Thus, while preventing the excreta from being directly guided to the movement portion, the excreta can be guided from the lateral side in the width direction of the testing member toward the non-skin side.

The color of the colored antibody disposed in the movement portion 66 may be color similar to the color of the colored region R20. With this configuration, the colored antibody can be concealed by the colored region R20 in the similar color, whereby the colored antibody can be made less noticeable. When comparing the color of the colored antibody disposed in the movement portion with the color of the colored region, the color of the colored antibody itself disposed in the movement portion, that is, the colored antibody that is not covered by the colored region, is compared with the color of the colored region. The similar color includes the same color. In the present specification, the color is "different" means that a hue differs by 5 or more (preferably 8 or more, more preferably 10 or more), a brightness differs by 1 or more (preferably 2 or more, more preferably 3 or more), or a chroma differs by 1 or more (preferably 2 or more, more preferably 3 or more) in the Munsell color system (100 hues). In the present specification, the color is "similar" or "similar color" means that the hue differs by less than 5, the brightness differs by less than 1, or the chroma differs by less than 1 in the Munsell color system (100 hues), including a case where the colors are the same. Alternatively, the color is "different" means that a color difference ΔE is 1 or more, preferably 2 or more, more preferably 3 or more, and even more preferably 5 or more. In this case, the color is "similar" or "similar color" means that the color difference ΔE is less than 1. A whitish color is a color having L* of 70 or more, preferably a color having L* of 80 or more, and more preferably a color having L* of 90 or more. However, the above-described color difference is measured as follows.

(1) X-Rite eXact Standard (measured diameter: 1.5 mm) manufactured by X. Rite, Incorporated is prepared.
(2) CIE L*a*b* coordinates of a portion to be measured in a sample are measured at 10 or more points while changing positions, and an average value thereof ([L1*, a1*, b1*]) is calculated.

(3) L*a*b* coordinates of a portion to be compared in the sample are measured at 10 or more points while changing positions, and an average value thereof ([L2*, a2*, b2*]) is calculated.

(4) The color difference ∆E is calculated by the following expression.

$$\Delta E = \sqrt{(L_1^* - L_2^*)^2 + (a_1^* - a_2^*)^2 + (b_1^* - b_2^*)^2}$$

A color difference between the color of the colored antibody disposed in the movement portion 66 and the color of the colored region R20 may be equal to or larger than 3.0. While an effect of concealing the colored antibody by the colored region R20 is obtained, the color of the colored antibody is different from the color of the colored region R20, so that the wearer can easily grasp a process in which the colored antibody moves during testing, and color development by a complex after the testing is completed. Thus, visibility of the testing result can be improved while preventing erroneous recognition before use.

A color difference between a region overlapped with the colored antibody in the thickness direction T in the colored region R20 and a region not overlapped with the colored antibody in the colored region R20 may be equal to or larger than 3.0. While an effect of concealing the colored antibody by the colored region R20 is obtained, the wearer can grasp the region where the colored antibody is arranged. The wearer can grasp the region where the colored antibody is arranged, and can carefully handle the absorbent article while grasping a portion that affects the testing, for example, by attaching the absorbent article so as not to touch the region where the colored antibody is arranged. Thus, testing accuracy can be prevented from being lowered.

The skin side T1 of the first indicator portion 62A and the second indicator portion 62B is not required to be covered by the colored region R20. That is, the colored region R20 may be arranged in a region that is not overlapped with the first indicator portion 62A and the second indicator portion 62B in the thickness direction T. With this configuration, it is possible to secure visibility of the first indicator portion 62A and the second indicator portion 62B from the skin side T1 of the absorbent article 1, and the wearer can grasp the testing result.

A brightness of the region where the colored region R20 is arranged on a surface on the skin side T1 of the absorbent article 1 may be lower than a brightness of the region where the first indicator portion 62A is arranged after color development. The brightness of the region where the colored region R20 is arranged is lower than the brightness of the region where the first indicator portion 62A is arranged, so that the first indicator portion 62A is less noticeable than the colored region R20 when seen from the skin side T1 of the absorbent article 1 after color development. Accordingly, while preventing the colored labeled antibody from being seen from the skin side T1 of the absorbent article before use of the absorbent article, the first indicator portion 62A can be made noticeable after color development, and the wearer can easily grasp the testing result. The brightness of the region where the colored region R20 is arranged on the surface on the skin side T1 of the absorbent article 1 may be lower than a brightness of the region where the second indicator portion 62B is arranged after color development. The brightness of the region where the colored region R20 is arranged is lower than the brightness of the region where the second indicator portion 62B is arranged, so that the second indicator portion 62B tends to be more noticeable than the colored region R20 when seen from the skin side of the absorbent article after color development. Accordingly, while preventing the colored labeled antibody from being seen from the skin side of the absorbent article before use of the absorbent article, the second indicator portion 62B can be made noticeable after color development, and the wearer can easily grasp the testing result.

In a modification, the brightness of the region where the colored region R20 is arranged on the surface on the skin side T1 of the absorbent article may be higher than the brightness of the region where the first indicator portion 62A is arranged after color development. According to the present aspect, the brightness of the region where the colored region R20 is arranged is higher than the brightness of the region where the first indicator portion 62A is arranged, so that the colored region R20 tends to be more noticeable than the first indicator portion 62A when seen from the skin side of the absorbent article after color development. The colored region R20 that does not cover the indicator portion is noticeable, so that the wearer can recognize the colored region R20 and the first indicator portion 62A to be distinguished from each other, and can easily grasp the testing result. The brightness of the region where the colored region R20 is arranged on the skin side surface of the absorbent article may be higher than the brightness of the region where the second indicator portion 62B is arranged after color development. According to the present aspect, the brightness of the region where the colored region R20 is arranged is higher than the brightness of the region where the second indicator portion 62B is arranged, so that the colored region R20 tends to be more noticeable than the second indicator portion 62B when seen from the skin side of the absorbent article after color development. The colored region R20 that does not cover the indicator portion is noticeable, so that the wearer can recognize the colored region R20 and the second indicator portion 62B to be distinguished from each other, and can easily grasp the testing result.

The brightness may be a brightness in the Munsell color system, and can be measured by a visual colorimetry method in which the brightness is visually determined by comparing with a color sample. Specifically, the visual determination can be made by comparing the color (hue, brightness, and chroma) of an object to be measured with a Munsell notation (color sample) on a visually equidistant numerical scale, using an expanded version of the Munsell system color chart issued by JAPAN COLOR ENTERPRISE Co., Ltd., for example.

The colored region R20 may be disposed on a liquid-impermeable sheet. With this configuration, the skin side T1 of the colored antibody is covered by the liquid-impermeable sheet. Thus, it is possible to prevent excreta from directly reaching the colored antibody side from the skin side T1 of the colored antibody. The excreta reach the region where the colored antibody is disposed after passing through a predetermined path (in the present embodiment, the contact portion). It is possible to prevent testing accuracy from being lowered due to the fact that a component other than the excreta component required for the testing reaches the region where the colored antibody is disposed, and improve the testing accuracy. In the present embodiment, the sheet on which the colored region R20 is disposed is the testing skin side sheet 68 that is liquid-impermeable. The testing skin side sheet 68 does not cover the skin side T1 of a partial region of the contact portion 64 and covers the movement portion 66 and the indicator portion 62. Thus, the excreta are guided to the movement portion 66 and the indicator portion 62 via the contact portion 64, and it is possible to prevent the excreta from directly reaching the movement portion and the indicator portion. The contact portion serves as a filter, and can guide, from the excreta, the excreta component required for the testing to the movement portion and the indicator portion.

The liquid-impermeable sheet on which the colored region R20 is disposed may include the high transmittance region R25 having a higher visible light transmittance than that of the colored region R20. The indicator portion 62 may be covered by the high transmittance region R25. The high transmittance region may be a region where the colored region is not disposed on the liquid-impermeable sheet. The indicator portion 62 is covered by the high transmittance region R25, so that visibility of the indicator portion 62 can be secured. By covering the skin side T1 of the indicator portion 62 by the liquid-impermeable sheet, it is possible to prevent excreta from directly reaching the indicator portion side from the skin side of the indicator portion. The high transmittance region R25 may cover at least part of the indicator portion 62, and preferably cover the entire indicator portion 62.

The colored region R20 may include a first colored region R21 that covers the colored antibody in the movement portion and a second colored region R22 that covers the skin side T1 of the absorbent portion 67. By covering the absorbent portion 67 by the second colored region R22, the colored absorbent portion 67 is difficult to be seen when the wearer sees the absorbent article 1 from the skin side T1. By causing the colored absorbent portion 67 to be difficult to be seen, the indicator portion 62 can be made relatively noticeable, and visibility of the testing result can be enhanced.

The first colored region R21 and the second colored region R22 may be arranged at an interval in the front-rear direction L. The indicator portion 62 may be arranged between the colored regions R20 in the front-rear direction L. By covering the movement portion 66 and the absorbent portion 67 by the colored region R20 and arranging the indicator portion 62 between the colored regions R20, the movement portion 66 and the absorbent portion 67 are concealed, the indicator portion 62 is made relatively noticeable, and the wearer can easily focus on the testing result.

By disposing the first colored region R21 and the second colored region R22 while holding the indicator portion 62 therebetween in the front-rear direction L, a range in which the testing result is indicated can be shown to the wearer. The first indicator portion 62A and the second indicator portion 62B are configured to develop color by capturing the complex and the labeled antibody, and to be difficult to be seen from the skin side T1 of the absorbent article 1 before color development. Thus, it may be difficult for the wearer to grasp the region where the testing result is indicated. However, by holding the indicator portion 62 between the first colored region R21 and the second colored region R22, the region where the testing result is indicated can be partitioned by the first colored region R21 and the second colored region R22, and the wearer can easily grasp the region where the testing result is indicated. An area of the first colored region R21 and an area of the second colored region R22 may be larger than a total area obtained by totaling an area of the first indicator portion 62A and an area of the second indicator portion 62B. By increasing the area of the first colored region R21 and the area of the second colored region R22, the region where the testing result is indicated can be made clearer. In the testing member 60 that is vertically long as in the present embodiment, a length in the front-rear direction L of the first colored region R21 and a length in the front-rear direction L of the second colored region R22 may be longer than a total length obtained by totaling a length in the front-rear direction L of the first indicator portion 62A and a length in the front-rear direction L of the second indicator portion 62B. By increasing the length in the front-rear direction L of the first colored region R21 and the length in the front-rear direction L of the second colored region R22, the region where the testing result is indicated can be made clearer.

The absorbent article 1 may include a first hydrophobic part 71 having hydrophobicity that covers the skin side T1 of the indicator portion 62 of the testing member. By covering the skin side T1 of the indicator portion 62 by the first hydrophobic part 71, it is possible to prevent excreta from directly reaching the indicator portion 62 from the skin side T1 of the indicator portion 62. Thus, it is possible to prevent visibility of the indicator portion 62 from being lowered due to excreta that has directly reached the indicator portion 62. The first hydrophobic part 71 may cover the skin side T1 of at least part of the indicator portion 62, but may preferably cover the skin side T1 of the entire indicator portion 62 (the first indicator portion 62A and the second indicator portion 62B).

The first hydrophobic part 71 in the present embodiment is constituted of a skin side cover sheet 18 and the testing skin side sheet 68. The first hydrophobic part 71 may be constituted of only one of the skin side cover sheet 18 and the testing skin side sheet 68. Both of the skin side cover sheet 18 and the testing skin side sheet 68 are liquid-impermeable sheets, and constitute the "skin side sheet" according to the present invention. That is, the skin side sheet covers the skin side T1 of the indicator portion 62. By covering the skin side T1 of the indicator portion 62 by the liquid-impermeable skin side sheet, it is possible to prevent excreta from directly reaching the indicator portion 62 from the skin side T1 of the indicator portion 62. The skin side cover sheet 18 is a sheet separated from the testing member 60, and located on the skin side T1 relative to the testing skin side sheet 68. The testing skin side sheet 68 is a sheet constituting the testing member 60. The testing skin side sheet 68 constitutes a "first skin side sheet" in the present invention, and the skin side cover sheet 18 constitutes a "second skin side sheet" in the present invention.

The hydrophobic part (including the first hydrophobic part 71, a second hydrophobic part 72, and a third hydrophobic part 73) may be a portion having low liquid permeability in which liquid permeability is reduced as compared with surroundings, or may be a portion having liquid impermeability in which liquid is not allowed to permeate. The hydrophobic part may be a portion to which a hydrophobic coating agent is applied, or a portion where a liquid-impermeable sheet is arranged. Examples of the hydrophobic coating agent include an adhesive agent (an HMA adhesive agent) and a liquid repellent (silicon), for instance. FIG. 3 illustrates ranges in the width direction of the first hydrophobic part 71, the second hydrophobic part 72, and the third hydrophobic part 73.

The first hydrophobic part 71 may cover at least the skin side T1 of the indicator portion 62, and may cover the skin side T1 of a region other than the indicator portion 62 in the testing member 60. The first hydrophobic part 71 may cover the skin side T1 of the movement portion 66. The skin side T1 of the movement portion is covered by the first hydrophobic part 71, so that it is difficult for excreta to directly reach the movement portion, whereby the testing accuracy can be improved. The first hydrophobic part 71 is not required to cover the skin side T1 of at least part of the contact portion 64. The first hydrophobic part 71 does not cover the skin side T1 of at least part of the contact portion 64, so that excreta is guided into the contact portion 64 via at least part of the contact portion 64, and guided to the movement portion 66 via the contact portion 64. The first hydrophobic part 71 covers the skin side T1 of the movement portion 66, so that it is possible to prevent excreta from directly reaching the movement portion 66. Thus, the excreta component required for the testing can be guided to the movement portion 66 via the contact portion 64, and the testing accuracy can be improved. By improving the testing accuracy, a correct testing result can be quickly indicated, and the user can easily grasp the testing result. The first hydrophobic part 71 may cover the skin side T1 of at least part of the movement portion 66, but may preferably cover the skin side T1 of the entire movement portion 66.

The first hydrophobic part 71 may be arranged to cover the skin side T1 of a portion other than the part of the contact portion 64. A configuration can be made such that excreta is brought into direct contact with only a predetermined region (part) of the contact portion 64, and a filter function of causing only required components to move toward the movement portion 66 side can be easily exhibited in the portion other than the part of the contact portion 64.

The skin side sheet (in the present embodiment, the skin side cover sheet 18) constituting the first hydrophobic part 71 may be arranged on the skin side T1 relative to the testing member 60, and arranged to straddle the contact portion 64, the movement portion 66, and the indicator portion 62. The skin side sheet straddles the contact portion 64, the movement portion 66, and the indicator portion 62, and is continuous from the contact portion 64 to the indicator portion 62. The first hydrophobic parts 71 are continuously disposed, so that it is possible to prevent excreta from infiltrating through a gap between the first hydrophobic parts 71 toward the testing member side, and prevent excreta from reaching the testing member via an unintended path. The skin side sheet constituting the first hydrophobic part 71 may cover part of the skin side of the contact portion 64 (does not necessarily cover the entire contact portion), preferably cover less than 50% of a total area of the contact portion, more preferably cover less than 20% of the total area of the contact portion, and even more preferably cover less than 10% of the total area of the contact portion.

As illustrated in FIG. 6, a hollow portion HP may be formed between the skin side sheet and the indicator portion 62. That is, the non-skin side surface of the skin side sheet and the skin side surface of the indicator portion 62 may be arranged at an interval in the thickness direction T. With this configuration, when a force is applied to the testing member 60 side from the skin side T1 of the absorbent article 1 due to a pressure during wearing, the hollow portion HP can absorb a pressure from the skin side T1. It is possible to prevent the indicator portion 62 from being collapsed due to the pressure during wearing, and the testing result can be indicated by the indicator portion 62. In the present embodiment, the absorbent portion 67 and the second colored region R22 are arranged between the membrane 66Y and the testing skin side sheet 68 in a region on a front side relative to the indicator portion 62, and the conjugate pad 66X and the sample pad 64Z are arranged between the membrane 66Y and the testing skin side sheet 68 in a region on a rear side relative to the indicator portion 62. Thus, the hollow portion HP is formed between the indicator portion 62 and the skin side sheet, and the indicator portion 62 is configured not to be pressed by the skin side sheet. Preferably, a thickness of the sample pad 64Z and a thickness of the conjugate pad 66X may be equal to or larger than 0.5 mm. The hollow portion HP is a space formed between members constituting the testing member. The hollow portion HP may be disposed in a region other than a region between the skin side sheet and the indicator portion 62 (between the testing skin side sheet 68 and the indicator portion).

As illustrated in FIG. 8, the hollow portion HP may be disposed between the sample pad 64Z and the testing non-skin side sheet 69, between the conjugate pad 66X and the colored region R20, and between the membrane 66Y and the testing skin side sheet 68.

Visible light transmittance of the skin side sheet constituting the first hydrophobic part 71 may be higher than visible light transmittance of the absorbent core 31. The visible light transmittance of the skin side sheet constituting the first hydrophobic part 71 is relatively high, so that it is possible to prevent the visibility of the testing member 60 from being lowered due to the presence of the skin side sheet. By covering the indicator portion 62 by the skin side sheet, it is possible to prevent the visibility of the testing member 60 from being lowered with high visible light transmittance while preventing the visibility of the indicator portion 62 from being lowered due to excreta that have directly reached from the skin side T1.

The absorbent article 1 may include the second hydrophobic part 72 having hydrophobicity that covers the non-skin side T2 of the indicator portion 62 of the testing member 60. The absorbent core 31 may be arranged on the non-skin side T2 relative to the second hydrophobic part 72. The second hydrophobic part 72 covers the non-skin side T2 of the indicator portion 62 and is arranged between the testing member 60 and the absorbent core 31, so that it is possible to prevent excreta absorbed by the absorbent core 31 from directly moving to the indicator portion 62. It is possible to prevent visibility of the indicator portion 62 from being lowered due to excreta absorbed by the absorbent core 31. The testing member 60 is located on the skin side T1 relative to the absorbent core 31, and the skin side T1 is not covered by the absorbent core 31. Thus, visibility of the indicator portion 62 can be easily secured when being seen from the skin side T1 of the absorbent article 1. The user can easily grasp the testing result. The second hydrophobic part 72 may cover the non-skin side T2 of at least part of the indicator portion 62, but may preferably cover the non-skin side T2 of the entire indicator portion 62 (the first indicator portion 62A and the second indicator portion 62B).

The second hydrophobic part 72 in the embodiment is constituted of a non-skin side cover sheet 19 and the testing non-skin side sheet 69. The second hydrophobic part 72 may be constituted of only one of the non-skin side cover sheet 19 and the testing non-skin side sheet 69. Both of the non-skin side cover sheet 19 and the testing non-skin side sheet 69 are liquid-impermeable sheets, and constitute the "non-skin side sheet" according to the present invention. That is, the non-skin side sheet covers the non-skin side T2 of the indicator portion 62. By covering the non-skin side T2 of the indicator portion 62 by the liquid-impermeable non-skin side sheet, it is possible to prevent excreta absorbed by the absorbent core 31 from moving to the indicator portion 62, and prevent the visibility of the indicator portion 62 from being lowered due to excreta moved from the absorbent core 31. The non-skin side cover sheet 19 is a sheet separated from the testing member 60, and located on the non-skin side T2 relative to the testing non-skin side sheet 69. The testing non-skin side sheet 69 is a sheet constituting the testing member 60. The testing non-skin side sheet 69 constitutes a "first non-skin side sheet" in the present invention, and the non-skin side cover sheet 19 constitutes a "second non-skin side sheet" in the present invention.

The second hydrophobic part 72 may cover at least the non-skin side T2 of the indicator portion 62, and may cover the non-skin side T2 of a region other than the indicator portion 62 in the testing member 60. The second hydrophobic part 72 may cover the non-skin side T2 of the contact portion 64 and the non-skin side T2 of the movement portion 66. The second hydrophobic part 72 covers the non-skin side T2 of the contact portion 64 and the non-skin side T2 of the movement portion 66, so that it is possible to prevent excreta absorbed by the absorbent core 31 from moving to the contact portion 64 and the movement portion 66. Thus, the excreta component required for the testing can be guided to the movement portion 66 via the contact portion 64, and the testing accuracy can be improved. By improving the testing accuracy, a correct testing result can be quickly indicated, and the user can easily grasp the testing result. The second hydrophobic part 72 may cover the non-skin side T2 of at least part of the movement portion 66 and the non-skin side T2 of at least part of the contact portion 64, but may preferably cover the non-skin side T2 of the entire movement portion 66 and the non-skin side T2 of the entire contact portion 64.

The non-skin side cover sheet 19 serving as the non-skin side sheet covers the non-skin side T2 of the testing member 60, and may be arranged while straddling the outer edge in the width direction W of the testing member 60. The non-skin side cover sheet 19 separate from the testing member 60 can prevent excreta from infiltrating into the testing member from the non-skin side of the testing member. The skin side cover sheet 18 serving as the skin side sheet covers the skin side T1 of the testing member 60, and may be arranged while straddling the outer edge in the width direction W of the testing member 60. The skin side cover sheet 18 separate from the testing member 60 can prevent excreta from infiltrating into the testing member 60 from the skin side T1 of the testing member 60.

Flexural rigidity of the non-skin side cover sheet 19 may be lower than flexural rigidity of the side region of the testing member 60. The flexural rigidity of the non-skin side cover sheet 19 that covers the non-skin side T2 of the testing member 60 is relatively low, so that a region on the non-skin side T2 relative to the testing member 60 is likely to be flexibly deformed, and hardness of the testing member 60 is enabled to be hardly felt. The flexural rigidity of the side region is relatively high, so that curling up of the testing member 60 can be prevented. The flexural rigidity of the non-skin side cover sheet 19 may be lower than the flexural rigidity of the testing non-skin side sheet 69. The non-skin side cover sheet 19 is arranged on the non-skin side T2 relative to the testing non-skin side sheet 69. The flexural rigidity of the non-skin side cover sheet 19 that covers the non-skin side T2 of the testing member 60 is relatively low, so that a region on the non-skin side T2 relative to the testing member 60 is likely to be flexibly deformed, and hardness of the testing member 60 is enabled to be more hardly felt.

Flexural rigidity of the skin side cover sheet 18 may be lower than the flexural rigidity of the side region of the testing member 60. The flexural rigidity of the skin side cover sheet 18 that covers the skin side T1 of the testing member 60 is relatively low, so that a region on the skin side T1 relative to the testing member 60 is likely to be flexibly deformed, and the hardness of the testing member 60 is enabled to be hardly felt. The flexural rigidity of the side region is relatively high, so that curling up of the testing member 60 can be prevented. The flexural rigidity of the skin side cover sheet 18 may be lower than the flexural rigidity of the testing skin side sheet 68. The skin side cover sheet 18 is arranged on the skin side T1 relative to the testing skin side sheet 68. The flexural rigidity of the skin side cover sheet 18 that covers the skin side T1 of the testing member 60 is relatively low, so that a region on the skin side T1 relative to the testing member 60 is likely to be flexibly deformed, and the hardness of the testing member 60 is enabled to be more hardly felt.

Herein, the flexural rigidity can be measured by a KES method. Specifically, measurement can be performed by using a bending tester KES-FB2-AUTO-A manufactured by KATO TECH CO., LTD., and samples of a size measurable for each of the cover sheet and the testing member are prepared. The flexural rigidity of the testing member is preferably obtained by cutting out the sample to include a portion including an antibody, and measuring rigidity of the portion including the antibody. Next, each sample is fixed between chucks of the bending tester so that the flexural rigidity in a lateral direction can be measured for each sample. The sample is bent to a front side up to a maximum curvature of +2.5 cm-1, then bent to a back side up to a maximum curvature of -2.5 cm-1, and returned to an original position thereafter. The flexural rigidity value is calculated from an average of a slope obtained when a slope of a bending moment with respect to curvature becomes substantially constant after starting bending to the front side, and a slope obtained when the slope of the bending moment with respect to the curvature becomes substantially constant after starting bending to the back side. Measurement is repeated five times for each sample, and an average value thereof is assumed to be the flexural rigidity.

The skin side sheet may come into contact with the non-skin side sheet at least on an outer side in the width direction with respect to the testing member 60. In the present embodiment, the skin side cover sheet 18 serving as the skin side sheet comes into contact with the non-skin side cover sheet 19 serving as the non-skin side sheet on the outer side in the width direction W with respect to the testing member 60. The skin side sheet and the non-skin side sheet being in contact with each other can prevent infiltration of excreta from the skin side T1, the non-skin side T2, and the lateral side of the testing member 60, and can prevent excreta from reaching the testing member via an unintended path. The skin side sheet and the non-skin side sheet may come into contact with each other at least on the outer side in the front-rear direction with respect to the testing member. In the present embodiment, the skin side cover sheet 18 and the non-skin side cover sheet 19 are in contact with each other on the outer side in the width direction W with respect to the testing member 60. The skin side cover sheet 18 and the non-skin side cover sheet 19 being in contact with each other can prevent infiltration of excreta from the skin side T1, the non-skin side T2, and the lateral side of the testing member 60, and can prevent excreta from reaching the testing member 60 via an unintended path. Preferably, the skin side cover sheet 18 may be joined with the non-skin side cover sheet 19 on the outer side in the width direction W with respect to the testing member 60. As the joining, joining by an adhesive agent and joining by welding such as heat welding can be exemplified. With this configuration, it is possible to further prevent infiltration of excreta from the skin side, the non-skin side, and the lateral side of the testing member 60, and further improve the testing accuracy.

In the width direction W, the outer edge of the skin side sheet may be displaced from the outer edge of the non-skin side sheet. That is, the outer edge of the skin side cover sheet 18 may be displaced from at least one of the outer edge of the non-skin side cover sheet 19 and the outer edge of the testing non-skin side sheet 69 in the width direction W. The outer edge of the testing skin side sheet 68 may be displaced from at least one of the outer edge of the non-skin side cover sheet 19 and the outer edge of the testing non-skin side sheet 69 in the width direction. The absorbent article 1 is held between the legs, and receives a force directed from the outer side toward the inner side in the width direction W. At this point, the outer edge of the non-skin side sheet is displaced from the outer edge of the skin side sheet in the width direction, and a plurality of deformation base points are disposed at intervals in the width direction W, so that the force applied from the outer side in the width direction W can be gradually reduced, a shape of the testing member 60 can be maintained, contact with the legs can be alleviated, and a sense of incongruity during wearing can be suppressed. The outer edge of the skin side cover sheet 18 may be displaced from the outer edge of the testing skin side sheet 68 in the width direction W, or the outer edge of the non-skin side cover sheet 19 may be displaced from the outer edge of the testing non-skin side sheet 69 in the width direction W. With this configuration, the outer edges of the sheets are arranged in a more stepped manner, and a larger number of deformation base points are disposed, whereby the force from the outer side in the width direction toward the testing member can be gradually reduced.

The outer end edge in the front-rear direction of the testing member 60 may be covered by at least one of the first non-skin side sheet (testing non-skin side sheet 69), the second non-skin side sheet (non-skin side cover sheet 19), the first skin side sheet (testing skin side sheet 68), and the second skin side sheet (skin side cover sheet 18). By covering the outer end edge of the testing member by any of the sheets, it is possible to suppress a sense of incongruity during wearing due to rigidity of the testing member. In the front-rear direction L, the outer end edge of the first non-skin side sheet, the outer end edge of the second non-skin side sheet, the outer end edge of the first skin side sheet, and the outer end edge of the second skin side sheet may be displaced from each other. At least one of the outer end edges of the four layers of sheets may be displaced, and preferably, all of the outer end edges of the respective sheets may be displaced from each other. The outer end edge of the first non-skin side sheet, the outer end edge of the second non-skin side sheet, the outer end edge of the first skin side sheet, and the outer end edge of the second skin side sheet are displaced from each other in the front-rear direction, so that the plurality of deformation base points may be disposed at intervals in the front-rear direction, and the absorbent article including the testing member can be arranged along a front-rear roundness of the body.

The absorbent article 1 may include the third hydrophobic parts 73 having hydrophobicity arranged on both sides in the width direction W of the testing member 60. The third hydrophobic part 73 is arranged on the outer side in the width direction with respect to the outer edge in the width direction W of the testing member 60, and prevents infiltration of excreta from the lateral side of the testing member 60. A length in the width direction W of each of the third hydrophobic parts 73 may be longer than the length in the width direction W of the testing member 60. The length in the width direction W of each of the third hydrophobic parts 73 is longer than the length in the width direction W of the testing member 60, so that infiltration of excreta from the lateral side of the testing member can be prevented over a wide range extending from the testing member 60 toward the outer side in the width direction W. Excreta is less likely to be arranged adjacent to the lateral side of the testing member, whereby it is possible to prevent visibility of the testing member from being lowered due to excreta accumulated on the lateral side of the testing member. The third hydrophobic part 73 in the embodiment is constituted of a portion extending toward the outer side in the width direction W relative to the testing member on the non-skin side cover sheet 19 and a portion extending toward the outer side in the width direction W relative to the testing member on the skin side cover sheet 18.

The skin side T1 of the testing member 60 may be covered by a sheet having lower breathability than that of the topsheet 11. The sheet having lower breathability than that of the topsheet 11 may have lower breathability than that of the topsheet 11, or may have no breathability. By covering the skin side of the testing member 60 by the sheet having lower breathability than that of the topsheet 11, the testing member 60 can be prevented from drying from the skin side T1 of the testing member 60. The testing member 60 can maintain appropriate humidity, and when excreta and an excreta component are guided thereto, the excreta component and the like can be smoothly moved, so that the testing can be appropriately performed. In the present embodiment, the skin side cover sheet 18 is arranged on the non-skin side T2 relative to the topsheet 11 and on the skin side T1 relative to the testing member 60. The skin side cover sheet 18 is constituted of a transparent resin film, and breathability of the skin side cover sheet 18 is lower than the breathability of the topsheet 11. The skin side cover sheet 18 constitutes a sheet having lower breathability than that of the topsheet 11. A length in the width direction and a length in the front-rear direction of the skin side cover sheet 18 are shorter than those of members arranged around the skin side cover sheet 18 (for example, the topsheet 11, the auxiliary sheet 15, or the absorbent core 31), and breathability of the members arranged around is higher than the breathability of the skin side cover sheet 18. The breathability of the members arranged around is high, so that fluidity and breathability of body fluid in the absorbent article can be secured.

As illustrated in FIG. 6, similarly to the testing skin side sheet, the skin side cover sheet 18 does not cover the skin side T1 of a partial region of the sample pad 64Z (a region that is not covered by the conjugate pad in the sample pad), and entirely covers the skin side T1 of the region covered by the conjugate pad 66X in the sample pad 64Z, the conjugate pad 66X, the membrane 66Y, and the absorbent portion 67. The skin side cover sheet 18 can also prevent excreta from directly reaching the movement portion 66 and the indicator portion 62. A width of the skin side cover sheet 18 is longer than a width of the testing member 60, and outer edges on both sides of the skin side cover sheet 18 may be located on the outer side in the width direction W than outer edges of the testing member 60. The skin side cover sheet 18 can prevent infiltration of excreta from the skin side and the lateral side of the testing member, and can prevent excreta from reaching the testing member 60 via an unintended path. In the width direction of the absorbent article, the outer edges on both sides of the skin side cover sheet 18 may be located on the outer side than the outer edges of the testing skin side sheet 68. The skin side cover sheet 18 can prevent infiltration of excreta from the skin side T1 and the lateral side of the testing member 60, and can prevent excreta from reaching the testing member 60 via an unintended path.

The skin side cover sheet 18 is arranged between the topsheet 11 and the testing member 60, and may be configured to be visually recognizable via the topsheet 11 from the skin side T1 of the absorbent article 1. By grasping the presence of the skin side cover sheet 18, the wearer can have an impression that the testing member 60 is protected, and can use the absorbent article 1 with a sense of security. With the skin side cover sheet 18 made of a resin film, when the absorbent article 1 is seen from the skin side T1 of the absorbent article 1, the skin side cover sheet 18 is likely to be noticeable due to diffuse reflection of light on the resin film. Thus, by focusing on the skin side cover sheet 18, the wearer can grasp the presence of the testing member 60 and the position of the testing member 60.

The testing member 60 is arranged on the skin side T1 relative to the absorbent core 31, and the absorbent article 1 may include the liquid-impermeable non-skin side cover sheet 19 arranged between the testing member 60 and the absorbent core 31. By covering the non-skin side T2 of the testing member 60 by the liquid-impermeable non-skin side cover sheet 19, it is possible to prevent excreta absorbed by the absorbent core 31 from moving to the indicator portion 62.

It is possible to prevent visibility of the indicator portion 62 from being lowered due to excreta absorbed by the absorbent core 31. Additionally, the required excreta component can be guided to the indicator portion 62 via the contact portion 64, and the testing accuracy can be improved.

The testing member 60 is arranged on the skin side T1 relative to the absorbent core 31, and the non-skin side T2 of the testing member 60 may be covered by a sheet having lower breathability than that of the absorbent core 31. The sheet having lower breathability than that of the absorbent core 31 may have lower breathability than that of the absorbent core 31, or may have no breathability. By covering the non-skin side T2 of the testing member 60 by the sheet having lower breathability than that of the absorbent core 31, the testing member 60 can be prevented from drying from the non-skin side T2 of the testing member 60. The testing member 60 can maintain appropriate humidity, and when excreta and an excreta component are guided thereto, the excreta component and the like can be smoothly moved, so that the testing can be appropriately performed. In the present embodiment, the non-skin side cover sheet 19 is constituted of a resin film, and breathability of the non-skin side cover sheet 19 is lower than breathability of the absorbent core 31. The non-skin side cover sheet 19 constitutes a sheet having lower breathability than that of the absorbent core 31. A length in the width direction and a length in the front-rear direction of the non-skin side cover sheet 19 are shorter than those of members arranged around the non-skin side cover sheet 19 (for example, the topsheet 11, the auxiliary sheet 15, or the absorbent core 31), and breathability of the members arranged around is higher than the breathability of the non-skin side cover sheet 19. The breathability of the members arranged around is high, so that fluidity and breathability of body fluid in the absorbent article can be secured.

Breathability of the backsheet 21 may be higher than breathability of at least one of the skin side cover sheet 18 and the non-skin side cover sheet 19. It is possible to achieve both an effect of preventing drying of the testing member by at least one of the skin side cover sheet 18 and the non-skin side cover sheet 19, and an effect of preventing steaminess after the absorbent core absorbs body fluid by the backsheet.

As illustrated in FIG. 6, the skin side cover sheet 18 may be displaced toward the rear side from the testing skin side sheet 68. The skin side cover sheet 18 may extend toward the front side with respect to a front end edge of the testing member 60. The non-skin side cover sheet 19 may be displaced toward the rear side from the testing non-skin side sheet 69. The non-skin side cover sheet 19 may extend toward the rear side with respect to a rear end edge of the testing member 60. The non-skin side cover sheet 19 covers the entire non-skin side T2 of the sample pad 64Z, the conjugate pad 66X, and the membrane 66Y. A width of the non-skin side cover sheet 19 is longer than the width of the testing member 60, and outer edges on both sides of the non-skin side cover sheet 19 may be located on the outer side in the width direction W than the outer edges of the testing member 60. The non-skin side cover sheet 19 can prevent infiltration of excreta from the non-skin side T2 and the lateral side of the testing member 60, and can prevent excreta from reaching the testing member 60 via an unintended path. In the width direction of the absorbent article, the outer edges on both sides of the non-skin side cover sheet 19 may be located on the outer side than the outer edges of the testing non-skin side sheet 69. The non-skin side cover sheet 19 can prevent infiltration of excreta from the non-skin side T2 and the lateral side of the testing member 60, and can prevent excreta from reaching the testing member 60 via an unintended path.

The testing member 60 in the present embodiment tests a health condition of the wearer based on vaginal discharge. As described above, vaginal discharge is preferable as a target for testing a health condition in excreta, but is difficult to be detected in some cases because a discharge amount thereof is small. In addition, vaginal discharge has high viscosity in excreta, and thus is less likely to reach the testing member 60, making it difficult to obtain an appropriate testing result in some cases. Thus, the topsheet 11 in the present embodiment is configured so as to appropriately test the health condition using vaginal discharge. Subsequently, the following describes the configuration of the topsheet 11 in detail.

The topsheet 11 is arranged on the skin side T1 relative to the testing member 60, and comes into contact with a skin surface of the wearer. Vaginal discharge excreted from the wearer is deposited on a skin surface of the topsheet 11, and permeates the topsheet 11 to be guided to the testing member 60 side. The topsheet 11 allows 6% or more of artificial body fluid, which has a viscosity of 15 to 30 Pa·s at 23°C (hereinafter, referred to as a predetermined viscosity) and is in contact with the skin side surface of the topsheet 11, to permeate to the non-skin side surface of the topsheet. As a result of extensive studies conducted by the applicant, it has been found that a general viscosity of vaginal discharge is 15 to 30 Pa·s at 23°C, and that the artificial body fluid having the predetermined viscosity exhibits a permeation aspect similar to that of the vaginal discharge. The topsheet 11 can allow the artificial body fluid having the predetermined viscosity, which is in contact with a surface on the skin side T1, to permeate to the non-skin side surface of the topsheet 11 at a high ratio of 6% or more. Accordingly, even vaginal discharge having higher viscosity than urine and menstrual blood can permeate the topsheet 11 to be guided to the testing member 60 side, and even a small amount of vaginal discharge can permeate the topsheet 11 at a high ratio to be guided to the testing member 60 side, whereby an appropriate testing result can be obtained based on the vaginal discharge. A permeation test for artificial body fluid for measuring artificial body fluid that permeates the topsheet will be described in detail later.

In a case where a sufficient amount of artificial body fluid is to be measured, the viscosity is measured as follows.

(1) The following equipment is prepared.

HAAKE RheoStress 1 manufactured by Thermo Fisher Scientific Inc. (sensor: Cone φ60 mm, 1° angle C60/1)

(2) An antibacterial liquid to be measured, in an amount of 38 ml, is poured into a container attached to the equipment, and a probe is immersed in the artificial body fluid.

(3) The viscosity is measured under the following conditions.

Temperature 23°C, humidity 60%, shear rate: 10.0 (l/s)

The topsheet 11 may be configured to allow 6% or more of the artificial body fluid, which has come into contact with the skin side surface of the topsheet 11, to permeate to the non-skin side surface of the topsheet 11 in a pressed state in which the skin side surface of the topsheet 11 is pressed toward the wearer side at a pressure of 1.60 to 1.70 kPa. Various forces are applied to the absorbent article 1 during wearing. As a result of extensive studies conducted by the applicant, it has been found that, in various scenes such as a scene in which the wearer changes a posture or a scene in which a wearable article to which the absorbent article 1 is attached is pulled up, the skin side surface of the topsheet 11 is in a pressed state in which it is pressed toward the wearer side at a pressure of at least 1.60 to 1.70 kPa. In this case, 6% or more of the artificial body fluid that has come into contact with the surface on the skin side T1 of the topsheet 11 is allowed to permeate to the non-skin side surface of the topsheet 11, so that the vaginal discharge that has permeated the topsheet 11 can be guided to the testing member 60 side, and an appropriate testing result can be obtained based on the vaginal discharge.

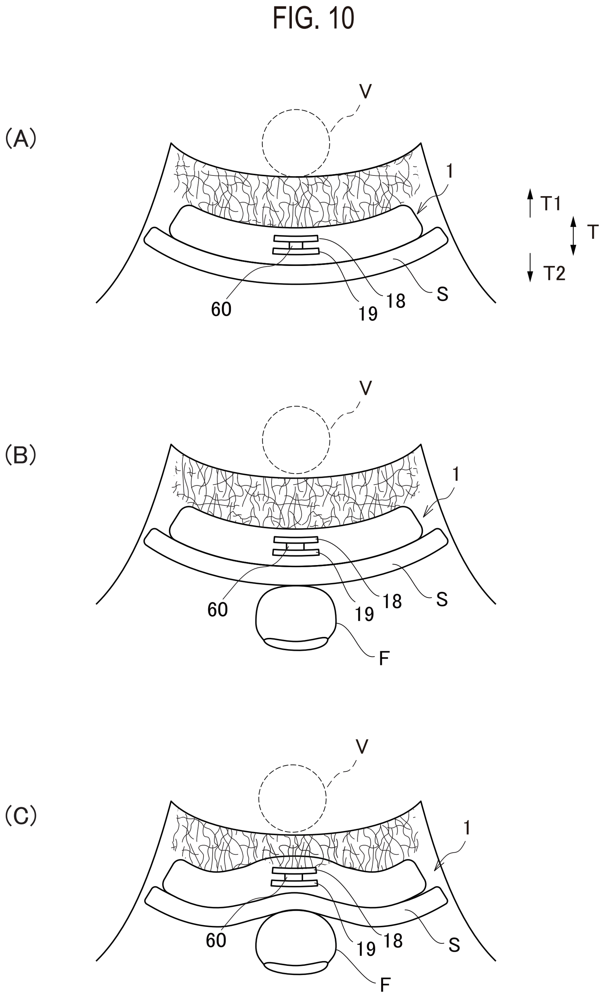

The absorbent article 1 may be an absorbent article that is used while being fastened to an inner surface of a wearable article S. The pressed state may be a state in which an outer surface of the wearable article S is pressed toward the wearer side so that the skin side surface of the topsheet 11 of the absorbent article 1 comes into contact with the skin surface of the wearer. FIG. 10 schematically illustrates a cross section along the width direction in a state in which the wearer is wearing the wearable article S to which the absorbent article 1 is attached. FIG. 10 illustrates a state in which the absorbent article 1 is pressed toward the wearer side, and a state in which the absorbent article 1 is not pressed toward the wearer side. In FIG. 10, for convenience of explanation, members other than the testing member 60, the skin side cover sheet 18, and the non-skin side cover sheet 19 in the absorbent article 1 are not illustrated. In FIG. 10, V schematically indicates a vaginal opening, S schematically indicates a wearable article, and F schematically indicates a finger of the wearer.

FIG. 10(A) illustrates an example of a state in which the wearable article S to which the absorbent article 1 is attached is not pressed toward the wearer side, that is, a normal wearing state. A panty liner, unlike a sanitary napkin, is intended to absorb a small amount of excreta, and is used on a daily basis rather than during a specific period as with a sanitary napkin. Thus, unlike a form in which a sanitary napkin is used together with sanitary shorts, it is often used while being attached to underwear for daily use. Accordingly, unlike a sanitary napkin that is pressed toward the wearer side by sanitary shorts, the panty liner is not pressed toward the wearer side, and thus a gap is likely to be generated between the wearer and the absorbent article 1, as illustrated in FIG. 10(A). Additionally, due to pubic hair around the vaginal opening V, a gap is likely to be generated between the wearer and the absorbent article 1.

To perform testing with the testing member 60, the wearer can intentionally press the outer surface of the wearable article S toward the wearer side so that the skin side surface of the topsheet 11 of the absorbent article 1 is brought into contact with the skin surface of the wearer. This pressing by the wearer is generally performed by pressing with a finger so as not to apply excessive force to a delicate genital area, and is performed so as to fill a gap between the skin side surface of the topsheet of the absorbent article 1 and the skin surface of the wearer. FIG. 10(B) illustrates a state in which a finger is placed on the non-skin side surface of the wearable article, and FIG. 10(C) illustrates a state in which the outer surface of the wearable article S is pressed toward the wearer side by the finger. As a result of extensive studies conducted by the applicant, it has been found that, when the wearer presses the outer surface of the wearable article S toward the wearer side with a finger, the topsheet 11 is in a pressed state in which it is pressed toward the wearer side at a pressure of at least 1.60 to 1.70 kPa. In the pressed state, by guiding vaginal discharge that has permeated the topsheet 11 to the testing member 60 side, an appropriate testing result can be obtained based on the vaginal discharge.

The topsheet 11 and the testing member 60 may be in contact with each other. At least part of the skin side surface of the testing member 60 may be in contact with the non-skin side surface of the topsheet 11. In the absorbent article 1 in the present embodiment, the skin side cover sheet 18 is arranged between the topsheet 11 and the testing member 60. The skin side cover sheet 18 does not cover part of the sample pad 64Z serving as the contact portion 64. Due to this, the topsheet 11 and the testing member 60 are in contact with each other in a region where the skin side cover sheet 18 is not arranged. With this configuration, vaginal discharge can be directly guided to the testing member 60 via the topsheet 11, and excreta can be quickly and securely guided to the testing member 60. To secure permeability of body fluid, it is preferable that no adhesive agent is applied to a portion where the topsheet 11 and the testing member 60 are in contact with each other.

The length in the width direction W of the testing member 60 may be shorter than a finger width of a female. When a finger is placed on the wearable article S along the front-rear direction L of the absorbent article to press it, a width of the finger is arranged along the width direction W of the testing member 60. When the length in the width direction W of the testing member 60 is shorter than the finger width of a female, the entire testing member 60 can be pressed with the finger. In this specification, the "finger width" does not mean a thickness of a finger, but specifically refers to the width of the finger in a nail spreading direction. In view of an operation of placing the finger on the outer surface of the wearable article to press it, the "finger width" may be at least one of a general middle finger width, index finger width, and ring finger width of a female, and is set to 10 mm, for example.

The length in the width direction W of the non-skin side cover sheet 19 that covers the non-skin side of the testing member 60 may be shorter than the finger width. With this configuration, when the finger is placed on the wearable article along the front-rear direction L of the absorbent article 1 to press it, the entire testing member 60 in the width direction W can be pressed with the finger via the non-skin side cover sheet 19. The length in the width direction W of the skin side cover sheet 18 that covers the skin side T1 of the testing member 60 may be shorter than the finger width. As illustrated in FIG. 10(C), when the outer surface of the wearable article S is pressed toward the wearer side, the skin side cover sheet 18 is arranged in a plane shape, and the testing member 60 is likely to be arranged so as to face an excretion opening along the plane. In a form in which the length in the width direction W of the skin side cover sheet 18 is longer than the testing member 60, the testing member 60 can be pressed so as to face the excretion opening throughout the entire width direction W of the testing member 60.

The testing member 60 may be arranged while straddling the center in the width direction W of the absorbent article 1. When the wearer places the finger on the wearable article to press it, the wearer tends to be aware of placing the wearable article on the excretion opening V and to position the finger so as to be located at the center in the width direction W of the absorbent article 1. Thus, the testing member, which is arranged while straddling the center in the width direction W of the absorbent article 1, can be pressed toward the wearer side by a pressing operation. The testing member 60 may have a vertically long shape in which the length in the front-rear direction L of the testing member 60 is longer than the length in the width direction W. When the finger is placed on the wearable article along the front-rear direction L of the absorbent article 1 to press it, the finger can be easily positioned along the testing member 60, a certain range extending in the front-rear direction L of the testing member 60 can be pressed with the finger, and the testing member 60 can be positioned closer to the body.

The topsheet 11 is constituted of a sheet containing fibers, and the average inter-fiber distance of the topsheet 11 may be longer than an average diameter of pubic hair of females. The average diameter of pubic hair may be an average diameter of that of adult females, which is specifically 90 $\mu$m. With this configuration, the average inter-fiber distance of the topsheet 11 is longer than the average diameter of pubic hair of females, so that pubic hair is likely to enter the topsheet 11 when the topsheet 11 comes into contact with the skin of the wearer. Vaginal discharge adhering to the pubic hair is likely to transfer to the topsheet 11. Preferably, the average inter-fiber distance on the skin side surface of the topsheet 11 may be longer than the average diameter of pubic hair of females. When the skin side surface of the absorbent article is pressed toward the wearer side, the pubic hair is more likely to enter the topsheet.

The contact portion 64 of the testing member 60 and the topsheet 11 are constituted of sheets containing fibers, and the average inter-fiber distance of the topsheet 11 may be longer than the average inter-fiber distance of the contact portion 64. Excreta discharged to the absorbent article 1 is guided to the contact portion 64 via the topsheet 11. Excreta in the topsheet 11 are likely to transfer to the contact portion 64 having a narrow average inter-fiber distance, and even a small amount of vaginal discharge is more likely to reach the testing member 60. A fiber density of the topsheet 11 may be higher than a fiber density of the contact portion 64. Excreta are likely to transfer from the topsheet 11 having a relatively high fiber density to the contact portion 64 having a relatively low fiber density, and even a small amount of vaginal discharge is more likely to reach the testing member 60.

The contact portion 64 may be in contact with the topsheet 11 in the uncompressed region. At least part of the contact portion 64 may be in contact with the topsheet 11 in the uncompressed region, and part of the contact portion may be in contact with the topsheet 11 in the compressed region. The topsheet 11 in the uncompressed region is not formed with irregularities by the compressed part 35 as compared with the topsheet 11 in the compressed region, and is likely to be brought into surface contact with the contact portion 64. Excreta in the topsheet 11 are likely to transfer to the contact portion 64, and even a small amount of vaginal discharge is more likely to reach the testing member 60.

The average thickness of the topsheet 11 in a region covering the contact portion 64 may be thicker than the average thickness of the topsheet 11 in a region covering the movement portion 66. The average thickness of the topsheet 11 covering the contact portion 64 is large, and a large capacity of a space for holding excreta can be secured. Thus, an amount of excreta drawn into the topsheet 11 in the region covering the contact portion 64 increases, and the excreta is likely to transfer from the topsheet 11 to the contact portion 64.

The non-skin side cover sheet 19 is a liquid-impermeable sheet, and may cover the entire region in the width direction W of the testing member 60. Outer edges on both sides of the non-skin side cover sheet 19 may extend toward the outer side in the width direction W with respect to the outer edges of the testing member 60. With this configuration, by covering the non-skin side T2 of the testing member 60 with the liquid-impermeable sheet over the entire region in the width direction W of the testing member 60, excreta that has reached the testing member 60 can be prevented from permeating from the testing member 60 to the non-skin side T2, and the excreta are likely to move in the testing member 60.

A contact angle of the artificial body fluid with respect to the topsheet 11 may be smaller than a contact angle of the artificial body fluid with respect to the skin of the wearer. Herein, wettability is evaluated with the "contact angle", and the smaller the contact angle, the more easily a surface is wetted and the more easily the artificial body fluid is drawn in. The contact angle is evaluated by using the artificial body fluid described above. As a result of extensive studies conducted by the applicant, it has been found that a PET plate exhibits hydrophilicity very similar to that of skin, and thus the contact angle with respect to the skin is assumed to be the contact angle with respect to the PET plate. The contact angle is measured as follows. (1) In a thermostatic chamber at 25°C, an automatic contact angle meter CA-V type (manufactured by Kyowa Interface Science Co., Ltd.), a plate made of polyethylene terephthalate (hereinafter, referred to as a "PET plate"), and a topsheet are prepared. The PET plate is PET-6010 (manufactured by C.I. TAKIRON Corporation, size: $41.5 \times 80.0 \times 1.0$ mm). (2) About 2 $\mu$L of a wiping property improving agent is dropped to each of the PET plate and the topsheet using a syringe, and 5 seconds after the dropping, a contact angle of the wiping property improving agent is measured by a $\theta/2$ method using the automatic contact angle meter CA-V type. (3) The measurement is performed ten times at different positions on each of the PET plate and the topsheet, and an average value thereof is adopted as the contact angle.

The testing member 60 in the present embodiment is a testing member using the immunochromatographic method for detecting a luteinizing hormone from vaginal discharge. The testing member used in the immunochromatographic method forms a complex through an antigen-antibody reaction with an excreta component, which is a component contained in excreta, develops color by an indication drug at a position where the complex has moved, and indicates a testing result. Thus, a required amount of excreta is larger than that for testing with test paper. However, according to the present invention, even a small amount of vaginal discharge can be guided to the testing member, and testing using the immunochromatographic method can be performed.

The absorbent article 1 may be folded along a folding line before use, and at least the indicator portion 62 of the testing member 60 may be arranged in a region that does not overlap with the folding line. A crease caused by the folding line is not formed on the indicator portion 62, and visibility of the indicator portion 62 can be secured. Preferably, the testing member 60 may be arranged in a region that does not overlap with the folding line. A crease caused by the folding line is not formed on the testing member 60, whereby the testing member 60 can be easily arranged along the body, and even a small amount of vaginal discharge can be easily guided to the testing member. The fastening portion 25 may be arranged in a region that overlaps with the testing member 60 in the thickness direction. The region in which the testing member 60 is arranged can be fastened to the wearable article via the fastening portion 25, whereby the testing member 60 can be pressed toward the wearer side via the wearable article, and even a small amount of vaginal discharge can be easily guided to the testing member.

The absorbent article is preferably configured so as not to inhibit transfer of excreta by an adhesive agent. As illustrated in FIG. 3, constituent elements constituting the absorbent article are joined to each other via an adhesive region to which an adhesive agent is applied. More specifically, a first adhesive region 551 is disposed between the topsheet 11 and the skin side cover sheet 18. The adhesive region is not disposed between the skin side cover sheet 18 and the testing member 60. A second adhesive region 552 is disposed between the testing member 60 and the non-skin side cover sheet 19. The adhesive region is not disposed between the non-skin side cover sheet 19 and the auxiliary sheet 15. A third adhesive region 553 is disposed between the auxiliary sheet 15 and the absorbent core 31. A fourth adhesive region 554 is disposed between the absorbent core 31 and the backsheet 21. As illustrated in FIG. 6, a non-adhesive region in which no adhesive agent is arranged is disposed on a surface where the movement portion 66, the indicator portion 62, the contact portion 64, and the absorbent portion 67 are in contact with each other. More specifically, the non-adhesive region is disposed on a surface where the movement portion 66 is in contact with the indicator portion 62, the non-adhesive region is disposed on a surface where the movement portion 66 is in contact with the contact portion 64, and the non-adhesive region is disposed on a surface where the movement portion 66 is in contact with the absorbent portion 67. According to the present aspect, no adhesive agent is arranged on the surface where the constituent elements are in contact with each other, so that it is possible to prevent inhibition of movement of the excreta component due to presence of the adhesive agent, and testing can be correctly and accurately performed.

Subsequently, the following describes a preferred method for manufacturing the testing member configured as described above. It should be noted that only part of steps of the following manufacturing method will be described, and as steps other than the described steps, steps in a known manufacturing method can be adopted. The manufacturing method includes at least a laminating step of arranging members constituting the movement portion, the indicator portion, and the absorbent portion between a testing non-skin side continuous sheet in which sheets constituting the testing non-skin side sheet 69 are continuous and a testing skin side continuous sheet in which sheets constituting the testing skin side sheet 68 are continuous, and a cutting step of cutting a laminated body laminated at the laminating step along an end edge in the second direction of the testing member. At the cutting step, the laminated body is pressed in the thickness direction T, and the laminated body is cut. According to this manufacturing method, in a process of cutting the laminated body, the end edge in the second direction of the testing member is pressed, the side region RS as an end in the second direction of the testing member is pressed more than the center region RC, and the thickness of the side region RS becomes thinner than the thickness of the center region RC. The side region RS is pressed more than the center region RC, whereby the density of the side region RS becomes higher than the density of the center region RC. Preferably, at the laminating step, the adhesive agent is not arranged on a surface where the contact portion 64 is in contact with the movement portion 66, a surface where constituent elements constituting the movement portion (the conjugate pad 66X and the membrane 66Y) are in contact with each other, a surface where the movement portion 66 is in contact with the indicator portion 62, and a surface where the movement portion 66 is in contact with the absorbent portion 67. At the cutting step, the laminated body may be thermally cut, and end edges in the second direction of the testing member may be joined by thermal cutting. The surface where the contact portion 64 is in contact with the movement portion 66, the surface where the constituent elements constituting the movement portion (the conjugate pad 66X and the membrane 66Y) are in contact with each other, the surface where the movement portion 66 is in contact with the indicator portion 62, and the surface where the movement portion 66 is in contact with the absorbent portion 67 are portions where excreta components move, and with this configuration, it is possible to prevent permeation of excreta from being inhibited by the adhesive agent. By joining the end edges in the second direction of the testing member by thermal cutting, the constituent elements can be joined without using the adhesive agent, and it is possible to prevent permeation of excreta from being inhibited by the adhesive agent. At the laminating step, in the step of configuring the movement portion, the indicator portion, and the absorbent portion between the testing non-skin side continuous sheet and the testing skin side continuous sheet, the movement portion, the indicator portion, and the absorbent portion in a state of being cut to have dimensions of the testing member may be arranged, or a continuous body in which the members constituting the movement portion, the indicator portion, and the absorbent portion are continuous may be arranged. In a form in which the laminated body includes the testing non-skin side continuous sheet, the testing skin side continuous sheet, and another continuous body, the continuous body may also be cut together at the cutting step. According to this manufacturing method, at the cutting step, the continuous body can be cut and joined together with the testing non-skin side continuous sheet and the testing skin side continuous sheet.

(3) Container according to embodiment

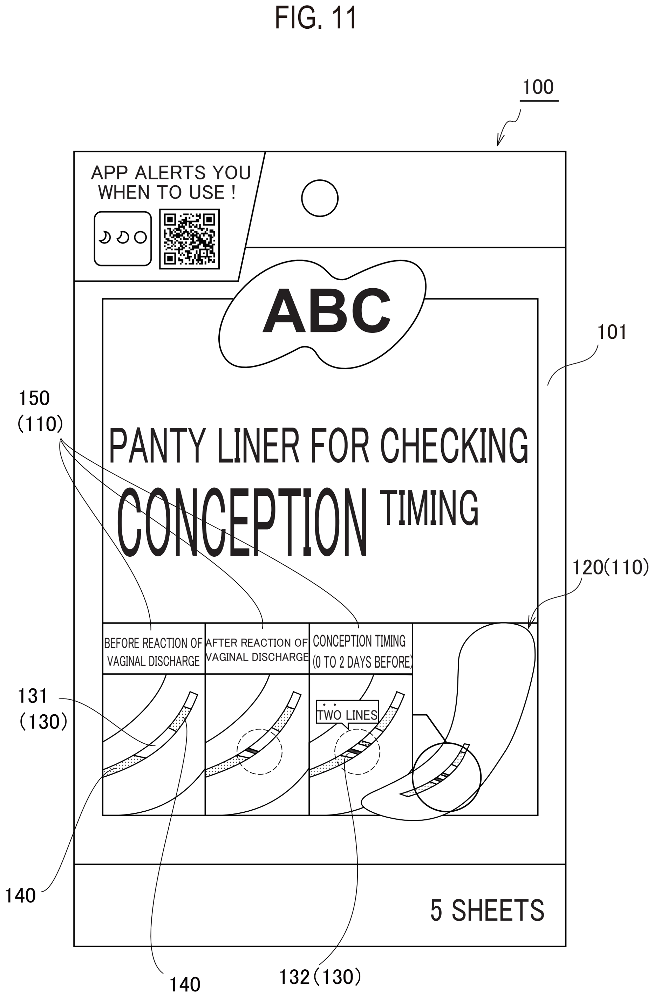

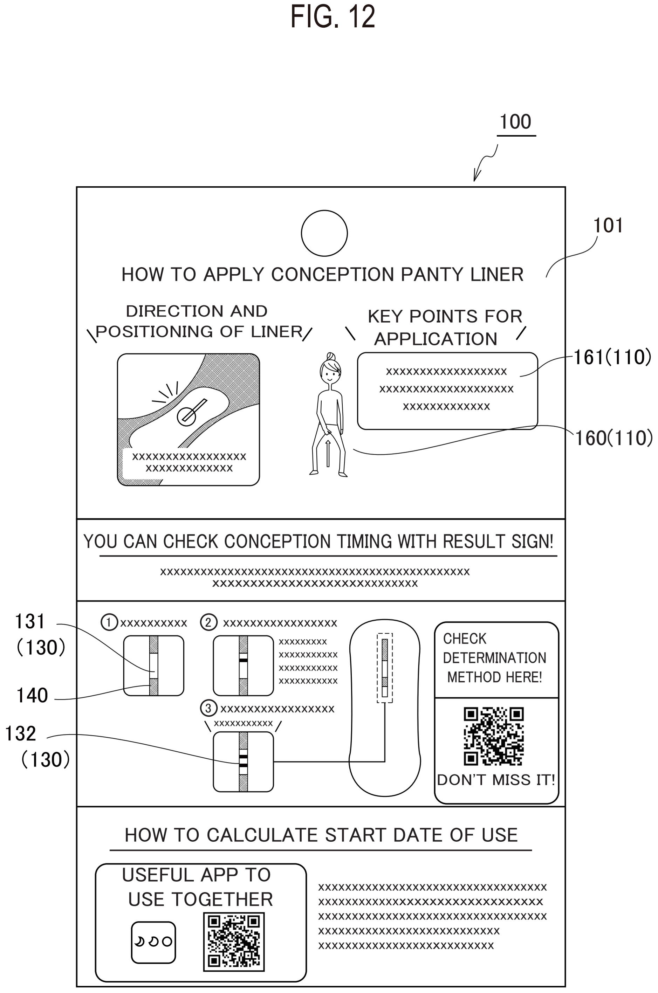

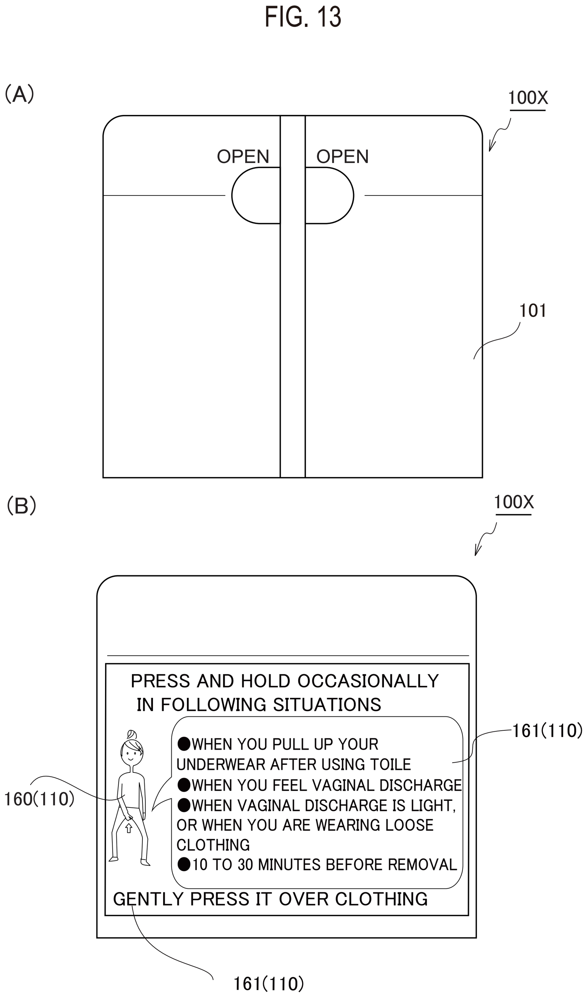

Subsequently, the following describes a container according to the embodiment (hereinafter, referred to as a container) 100. In the following description, some of the elements that are the same as those in the embodiment described above will be denoted by the same reference numerals, and description thereof will be omitted. FIG. 11 is a front view of the container 100 according to a first mode, and FIG. 12 is a back view of the container 100 according to the first mode. The container 100 includes the absorbent article 1 and a container main body 101 for containing the absorbent article. The container main body 101 may be a packaging body 100X (refer to FIG. 13) that individually packages the absorbent article 1, or may be a container main body that contains a plurality of absorbent articles or packaging bodies. The container 100 according to the first mode contains a plurality of packaging bodies 100X, each of which is the absorbent article individually packaged with a packaging sheet. FIG. 13 illustrates a container 100X according to a second mode, in which the packaging body that individually packages the absorbent article 1 constitutes the container, and the packaging sheet constitutes the container main body. FIG. 13(A) is a front view of the container 100X, and FIG. 13(B) is a back view of the container 100X. The container main body 101 may be constituted of paper, or may be constituted of a resin film. Preferably, the container main body (packaging sheet) of the container 100X may be constituted of an aluminum laminate material (PET12/AL6/CPP30) to suppress deterioration of the testing member before use.

The container main body 101 includes a design portion 110 that is visually recognizable from an outer surface of the container. The design portion 110 may be configured by printing, or may be configured by a seal attached to a base material (for example, a resin film) constituting the container main body 101. The design portion 110 may include an article design portion 120 indicating the absorbent article. The article design portion 120 may indicate at least part of the absorbent article, may indicate the whole of the absorbent article 1, or may indicate part of the absorbent article 1 such as a portion including the testing member 60. The article design portion 120 may include an indicator design portion 130 indicating the indicator portion 62 of the testing member 60, and a colored design portion 140 indicating the colored region. The wearer sees the article design portion 120 attached to the outer surface of the container at the time of using or purchasing the absorbent article 1. The article design portion 120 includes the indicator design portion 130 and the colored design portion 140. Accordingly, the wearer can grasp the function of the absorbent article 1 (including the testing member 60), and the state of the testing member 60 (state of being covered by the colored region R20) before use. The wearer can grasp the state of the testing member 60 of the absorbent article 1 before use, so that it is possible to prevent erroneous recognition such that a reaction has occurred before use when the wearer sees the actual absorbent article 1.

The indicator design portion 130 may include a pre-indicator design portion 131 indicating the indicator portion before color development, and a post-indicator design portion 132 indicating the indicator portion after color development. The post-indicator design portion 132 may indicate at least the first indicator portion 62A after color development, and may preferably indicate both of the first indicator portion 62A after color development and the second indicator portion 62B after color development. With this configuration, a state of the indicator portion before color development and a state of the indicator portion after color development can be shown to the wearer, and the wearer can understand a function, a usage method, and the like of the testing member and can use the absorbent article with a sense of security.

The post-indicator design portion 130 may include a first post-indicator design portion indicating a state in which an appropriate color developing reaction is exhibited, and a second post-indicator design portion indicating the indicator portion after inappropriate color development. The first post-indicator design portion indicates a state in which an appropriate color developing reaction is exhibited, and specifically indicates a state in which both the first indicator portion and the second indicator portion have developed color. The second post-indicator design portion indicates a state in which an inappropriate color developing reaction is exhibited, and specifically, examples thereof may include: a state in which the first indicator portion has developed color and the second indicator portion has not developed color; a state in which the entire indicator portion including the first indicator portion and the second indicator portion has developed color; and a state in which the center in the width direction of the indicator portion has developed color and a side part in the width direction of the indicator portion has not developed color. With this configuration, the appropriate color developing state and the inappropriate color developing state can be shown to the wearer by the article design portion, and the wearer can understand a function, a usage method, and the like of the testing member and can use the absorbent article with a sense of security.

The design portion 110 of the container main body 101 may include a testing explanation portion 150 including at least one of a method of using the testing member, a function of the testing member, and a method of discriminating a testing result. The testing explanation portion may be characters or illustrations. By providing the testing explanation portion, the wearer can better understand the function, the usage method, and the like of the testing member, and can use the absorbent article with a sense of security.

The design portion 110 may include a pressing indicator portion 160. The pressing indicator portion 160 indicates a state in which the outer surface of the wearable article S is pressed toward the wearer side to bring the skin side surface of the topsheet 11 of the absorbent article 1 into contact with the skin surface of the wearer. As illustrated in FIG. 12 and FIG. 13, the pressing indicator portion 160 in the present embodiment is an illustration attached to a back surface of the container 100 and a back surface of the container 100X, and indicates a state in which the wearer places a finger on an outer side of a garment and presses an outer surface of the wearable article from the garment side toward the wearer. By seeing the pressing indicator portion 160, the wearer can grasp the state in which the outer surface of the wearable article S is pressed toward the wearer so that the skin side surface of the topsheet 11 of the absorbent article 1 is brought into contact with the skin surface of the wearer, and the pressed state can be easily performed. When the wearer performs the pressed state, vaginal discharge can be easily guided to the testing member 60, and an appropriate testing result can be obtained based on the vaginal discharge.

The design portion 110 may include, in addition to the pressing indicator portion 160, an operation explanation portion 161 including at least one of explanation of a pressing operation of pressing the outer surface of the wearable article toward the wearer side and explanation of prompting the wearer to perform the pressing operation. The wearer can easily understand the pressing operation through the operation explanation portion 161, and when the wearer performs the pressed state, vaginal discharge can be easily guided to the testing member, and an appropriate testing result can be obtained based on the vaginal discharge.

(4) Sheet for absorbent article

The present invention may be a sheet for the absorbent article that is preferably used for the absorbent article. The sheet for the absorbent article allows 6% or more of the artificial body fluid, which has the predetermined viscosity (viscosity of 15 to 30 Pa·s at 23°C) and is in contact with the skin side surface of the sheet for the absorbent article, to permeate to the non-skin side surface of the sheet for the absorbent article. For the sheet for the absorbent article, the configuration of the topsheet in the absorbent article described above can be adopted. The sheet for the absorbent article can be used particularly preferably in the absorbent article including the testing member. The sheet for the absorbent article according to the present aspect can allow the artificial body fluid having the predetermined viscosity, which is in contact with the skin side surface, to permeate to the non-skin side surface of the sheet at a high ratio of 6% or more. Accordingly, even vaginal discharge having a higher viscosity than that of urine and menstrual blood can be allowed to permeate the sheet. Particularly, by using the sheet for the absorbent article on the skin side of the testing member, it is possible to allow vaginal discharge to permeate the sheet at a high ratio to be guided to the testing member side, whereby the health condition of the wearer can be appropriately tested based on the vaginal discharge.

It is preferable that the sheet for the absorbent article and the topsheet 11 constituting the absorbent article in the above embodiment do not have water repellency. More specifically, the sheet for the absorbent article and the topsheet 11 are not required to be subjected to water-repellent treatment. A water-repellent treatment method is not particularly limited, and a normal method can be widely adopted. Specifically, examples thereof include a method of using a water-repellent treatment agent such as silicon oil, fluororesin, or methylhydrogenpolysiloxane, or an oil agent containing these components. The sheet for the absorbent article and the topsheet 11 may be subjected to hydrophilic treatment. The hydrophilic treatment may include, for example, a hydrophilic oil agent. The body fluid can be more easily drawn in since the sheet is not subjected to water-repellent treatment, and the body fluid can be easily drawn in since the sheet is subjected to hydrophilic treatment.

Examples of a preferable configuration of the topsheet 11 include an air-through nonwoven fabric, a spunbond nonwoven fabric, an SMS nonwoven fabric, and a spunlace nonwoven fabric. In the topsheet 11, an opening penetrating in the thickness direction may be formed. In a permeation test described later, the topsheet 11 may allow 6% or more of the artificial body fluid to permeate, preferably allow 10% or more thereof to permeate, and more preferably allow 14% or more thereof to permeate. As a more preferred nonwoven fabric in the present embodiment, it is possible to exemplify a spunbond nonwoven fabric made of polypropylene fibers having a thickness equal to or larger than 0.1 mm and equal to or smaller than 0.2 mm, and a basis weight of fibers equal to or larger than 8 g/m$^2$ and equal to or smaller than 12 g/m$^2$.

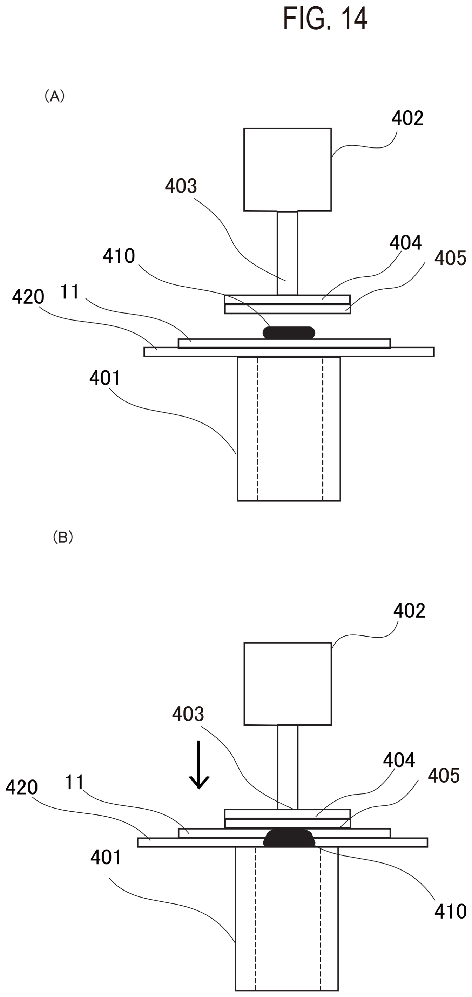

Subsequently, the following describes the permeation test for the artificial body fluid based on FIG. 14. FIG. 14 schematically illustrates a testing device for the permeation test.

As the artificial body fluid in the present invention, it is possible to use corn starch (example: Fuekinori Kogyo Co., Ltd.) adjusted to have a viscosity of 15 to 30 Pa·s at 23°C.

The testing device includes: a cylindrical jig 401 on which a topsheet (in measurement of the sheet for the absorbent article, the sheet for the absorbent article; the same applies hereinafter) is placed; a digital force gauge (manufactured by SHIMPO (NIDEC DRIVE TECHNOLOGY CORPORATION)) 402; a force gauge stand for supporting the digital force gauge (manufactured by SHIMPO (NIDEC DRIVE TECHNOLOGY CORPORATION)) (not illustrated); a flat plate (acrylic plate) 404 attached to a distal end 403 of the digital force gauge 402; and an artificial leather (PBZ13001 of IDEATEX JAPAN, (Katsutoku Building, 2-25-6, Taito, Taito-ku, Tokyo)) 405 sticked to a surface on the topsheet 11 side of the flat plate 404. The testing device is set in advance. Specifically, filter paper 420 having dimensions to cover a hollow portion of the cylindrical jig 401 is prepared, and a weight of the filter paper is measured. As the filter paper, qualitative filter paper No. 2 having a size of 100 mm × 100 mm (ADVANTEC TOYO KAISHA, LTD.) can be used. Subsequently, the filter paper 420 is arranged to cover the hollow portion of the jig 401, and fixed to the filter paper 420. Subsequently, the topsheet 11 is arranged on the filter paper 420 so that the skin side surface of the topsheet 11 faces upward, and the topsheet 11 is fixed to the jig and the filter paper. For example, both ends of the topsheet 11 are fixed with cellophane tape. After the digital force gauge 402 is fixed to the force gauge stand, the acrylic plate (30 mm × 30 mm) 404 is fixed to the distal end 403 of the digital force gauge 402, and the artificial leather (30 mm × 30 mm) 405 is further fixed onto the acrylic plate. The topsheet 11 for performing the permeation test is prepared. The topsheet 11 is peeled off from the absorbent article, and only the topsheet 11 is taken out. At this point, if the topsheet cannot be peeled off due to an adhesive agent, the topsheet is cooled (for example, the topsheet is frozen with liquid nitrogen, and then air-dried until components of the liquid nitrogen volatilize from the taken-out topsheet), and the topsheet 11 is taken out. Dimensions of the topsheet are assumed to be dimensions on the cylindrical jig (equal to or larger than dimensions of the cylindrical jig).

The permeation test is started 10 seconds after 0.5 mg of artificial body fluid is dropped onto the skin side surface of the topsheet 11. FIG. 14(A) illustrates a state in which artificial body fluid 410 is dropped onto the topsheet 11 disposed on the jig. Subsequently, the digital force gauge 402 is lowered at 100 mm/min, and lowering of the digital force gauge is stopped under a condition in which the digital force gauge being in contact with the topsheet 11 indicates a numerical value of 2.16 kPa. FIG. 14(B) illustrates a state in which lowering of the digital force gauge is stopped. The topsheet 11 and the filter paper 420 are removed from the jig, and the weight of the filter paper is measured. A value obtained by subtracting the weight of the filter paper before the permeation test from the weight of the filter paper after the permeation test is the weight of the artificial body fluid that has permeated the topsheet 11. The permeation test is performed five times using five samples, and an average value thereof is used.

The present invention has thus been explained in detail by using the above embodiment. It is, however, apparent to persons skilled in the art that the present invention is not limited to the embodiment described in the present specification. It is possible to carry out the present invention in revised or modified aspects, without departing from the gist and the scope of the present invention defined by the patent claims. Consequently, the description of the present specification aims to explain certain examples and is not intended to restrict the present invention.

The entire content of Japanese Patent Application No. 2023-083677, filed on May 22, 2023, is incorporated herein by reference.

[INDUSTRIAL APPLICABILITY]

It is possible to provide an absorbent article with which a wearer can easily see a testing result of a testing member.

[REFERENCE SIGNS LIST]

1: absorbent article
11: topsheet
15: auxiliary sheet
18: skin side cover sheet (second skin side sheet)
19: non-skin side cover sheet (second non-skin side sheet)
21: backsheet
31: absorbent core
35: compressed part
60: testing member
62: indicator portion
62A: first indicator portion
62B: second indicator portion
64: contact portion
64Z: sample pad
66: movement portion
66X: conjugate pad
66Y: membrane
67: absorbent portion
68: testing skin side sheet (first skin side sheet)
69: testing non-skin side sheet (first non-skin side sheet)
71: first hydrophobic part
72: second hydrophobic part
73: third hydrophobic part
100: container
101: container main body
110: design portion
120: article design portion
130: indicator design portion
131: pre-indicator design portion
132: post-indicator design portion
140: colored design portion
160: pressing indicator portion
RC: center region
RS: side region
R20: colored region
R21: first colored region
R22: second colored region
R25: high transmittance region
HP: hollow portion
S1: front-side region
S2: rear-side region
S3: crotch region
L: front-rear direction
T: thickness direction
T1: skin side
T2: non-skin side
W: width direction

**Claims**

1. An absorbent article comprising a testing member for testing a health condition of a wearer, wherein

the testing member includes an indicator portion that develops color based on an excreta component as a component contained in excreta,
the absorbent article includes a first hydrophobic part having hydrophobicity that covers a skin side of the indicator portion of the testing member, and
the indicator portion after color development is visually recognizable from a skin side of the absorbent article.

2. The absorbent article according to claim 1, wherein

   the testing member includes a contact portion that comes into contact with excreta, and a movement portion that guides, to the indicator portion, an excreta component as a component contained in excreta guided from the contact portion, and
   the first hydrophobic part covers the skin side of the movement portion.

3. The absorbent article according to claim 2, wherein

   the first hydrophobic part is constituted of a liquid-impermeable skin side sheet, and
   the skin side sheet is arranged on the skin side relative to the testing member, and arranged to straddle the contact portion, the movement portion, and the indicator portion.

4. The absorbent article according to claim 1 or 2, wherein

   the absorbent article includes an absorbent core,
   the first hydrophobic part is constituted of a liquid-impermeable skin side sheet, and
   a visible light transmittance of the skin side sheet is higher than a visible light transmittance of the absorbent core.

5. The absorbent article according to claim 1 or 2, wherein

   the absorbent article includes an absorbent core, and
   the testing member is arranged on the skin side relative to the absorbent core.

6. The absorbent article according to claim 1 or 2, wherein

   the absorbent article includes a second hydrophobic part having hydrophobicity that covers a non-skin side of the indicator portion of the testing member, and
   an absorbent core is arranged on the non-skin side relative to the second hydrophobic part.

7. An absorbent article comprising: a testing member for testing a health condition of a wearer; and an absorbent core, wherein

   the testing member includes an indicator portion that develops color based on an excreta component as a component contained in excreta,
   the absorbent article includes a second hydrophobic part having hydrophobicity that covers a non-skin side of the testing member,
   the absorbent core is arranged on the non-skin side relative to the second hydrophobic part, and
   the indicator portion after color development is visually recognizable from a skin side of the absorbent article.

8. The absorbent article according to claim 7, wherein

   the testing member includes a contact portion that comes into contact with excreta, and a movement portion that guides, to the indicator portion, an excreta component as a component contained in excreta guided from the contact portion, and
   the second hydrophobic part covers the non-skin side of the contact portion and the non-skin side of the movement portion.

9. The absorbent article according to claim 1 or 7, wherein
   the testing member is visually recognizable from the skin side of the absorbent article before color development of the indicator portion, and is visually unrecognizable from the non-skin side of the absorbent article.

10. The absorbent article according to claim 1 or 7, wherein

    the absorbent article includes third hydrophobic parts having hydrophobicity that are arranged on both sides in a width direction of the testing member, and
    a length in the width direction of each of the third hydrophobic parts is longer than a length in the width direction of the testing member.

**11.** The absorbent article according to claim 1 or 7, comprising:

a first hydrophobic part having hydrophobicity that covers a skin side of the indicator portion of the testing member, and a second hydrophobic part having hydrophobicity that covers a non-skin side of the indicator portion of the testing member, wherein
the first hydrophobic part is constituted of a liquid-impermeable skin side sheet,
the second hydrophobic part is constituted of a liquid-impermeable non-skin side sheet, and
the skin side sheet and the non-skin side sheet are in contact with each other at least on an outer side in a width direction relative to the testing member.

**12.** The absorbent article according to claim 11, wherein
an outer edge of the skin side sheet is displaced from an outer edge of the non-skin side sheet in the width direction.

**13.** The absorbent article according to claim 1 or 7, wherein

the absorbent article includes a topsheet located on the skin side relative to the testing member, and a backsheet located on the non-skin side relative to the testing member,
the topsheet and the backsheet are constituted of sheets containing fibers, and
an average inter-fiber distance of the backsheet is shorter than an average inter-fiber distance of the topsheet.

**14.** The absorbent article according to claim 1 or 7, wherein
the testing member is arranged to be inclined with respect to a front-rear direction of the absorbent article in a plan view of the absorbent article.

**15.** The absorbent article according to claim 1 or 7, wherein
a projecting part projecting toward an outer side in a width direction of the absorbent article and a recessed part recessed toward an inner side in the width direction with respect to the projecting part are disposed on an outer portion of the testing member.

**16.** The absorbent article according to claim 1 or 7, wherein

the absorbent article includes a topsheet located on a skin side relative to the testing member, and a compressed part obtained by compressing at least the topsheet in a thickness direction,
the compressed part includes a first compressed part and a second compressed part arranged on opposite sides across the testing member in a width direction of the absorbent article, and
a distance in the width direction between the first compressed part and the testing member is different from a distance in the width direction between the second compressed part and the testing member.

**17.** The absorbent article according to claim 1 or 7, wherein
at a cross section along a front-rear direction of the absorbent article, a skin side surface of the testing member is inclined toward the non-skin side from the center in the front-rear direction of the testing member toward an outer end of the testing member.

# FIG. 1

# FIG. 2

# FIG. 3

# FIG. 4

# FIG. 5

# FIG. 6

# FIG. 7

# FIG. 8

(A)

64Z(64)    HP

RS    RC    RS

T1  T

T2

69,72    19,72

(B)

68,71

R21(R20)

HP    RC    18,71    RS

RS

66X(66)

66Y(66)

69,72    19,72

(C)

HP  18,71    68,71

RS    RC    RS

66Y(66)

69,72    19,72

(A)    (B)    (C)

FIG. 9

EP 4 710 903 A1

41

# FIG. 10

(A)

(B)

(C)

# FIG. 11

100

APP ALERTS YOU
WHEN TO USE !

**ABC**

101

150
(110)

PANTY LINER FOR CHECKING
CONCEPTION TIMING

120(110)

| BEFORE REACTION OF VAGINAL DISCHARGE | AFTER REACTION OF VAGINAL DISCHARGE | CONCEPTION TIMING (0 TO 2 DAYS BEFORE) | |
|---|---|---|---|

TWO LINES

131
(130)

140

5 SHEETS

140

132(130)

# FIG. 12

100

101

## HOW TO APPLY CONCEPTION PANTY LINER

### DIRECTION AND \POSITIONING OF LINER/

xxxxxxxxxxxxxxxxx
xxxxxxxxxxxxx

### KEY POINTS FOR APPLICATION

xxxxxxxxxxxxxxxxxx
xxxxxxxxxxxxxxxxxx
xxxxxxxxxxxx

161(110)

160(110)

## YOU CAN CHECK CONCEPTION TIMING WITH RESULT SIGN!

xxxxxxxxxxxxxxxxxxxxxxxxxxxxxxxxxxxxxxxxxxxxx
xxxxxxxxxxxxxxxxxxxxxxxxxxxx

131
(130)

140

132
(130)

① xxxxxxxxxx    ② xxxxxxxxxxxxxxx

xxxxxxxxx
xxxxxxxxxx
xxxxxxxxxx
xxxxxxxxxx

③ xxxxxxxxxxxxxxxx
\xxxxxxxxxxx/

CHECK
DETERMINATION
METHOD HERE!

DON'T MISS IT!

## HOW TO CALCULATE START DATE OF USE

### USEFUL APP TO USE TOGETHER

xxxxxxxxxxxxxxxxxxxxxxxxxxxxxxx
xxxxxxxxxxxxxxxxxxxxxxxxxxxxx
xxxxxxxxxxxxxxxxxxxxxxxxxxxxx
xxxxxxxxxxxxxxxxxxxxxxxxxxx
xxxxxxxxxxxxxxxxxxxxxxxxx

# FIG. 13

(A)

100X

OPEN     OPEN

101

(B)

100X

PRESS AND HOLD OCCASIONALLY
IN FOLLOWING SITUATIONS

●WHEN YOU PULL UP YOUR
UNDERWEAR AFTER USING TOILE
●WHEN YOU FEEL VAGINAL DISCHARGE
●WHEN VAGINAL DISCHARGE IS LIGHT,
OR WHEN YOU ARE WEARING LOOSE
CLOTHING
●10 TO 30 MINUTES BEFORE REMOVAL

GENTLY PRESS IT OVER CLOTHING

161(110)

160(110)

161(110)

# FIG. 14

(A)

(B)

## INTERNATIONAL SEARCH REPORT

| International application No. |
|---|
| **PCT/JP2024/018735** |

**A. CLASSIFICATION OF SUBJECT MATTER**

*A61F 13/42*(2006.01)i; *A61F 13/84*(2006.01)i
FI: A61F13/42 B; A61F13/84

According to International Patent Classification (IPC) or to both national classification and IPC

**B. FIELDS SEARCHED**

Minimum documentation searched (classification system followed by classification symbols)

A61F13/42; A61F13/84

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-22585 A (UNI-CHARM CO., LTD.) 13 February 2020 (2020-02-13) paragraphs [0047]-[0077], fig. 1-7 | 1-17 |
| Y | JP 2020-22584 A (UNI-CHARM CO., LTD.) 13 February 2020 (2020-02-13) paragraphs [0083]-[0084], fig. 7 | 1-17 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **24 July 2024** | **06 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/018735**

| Patent document cited in search report | | | Publication date (day/month/year) | Patent family member(s) | | | Publication date (day/month/year) |
|---|---|---|---|---|---|---|---|
| JP | 2020-22585 | A | 13 February 2020 | CN | 112512471 | A | |
| JP | 2020-22584 | A | 13 February 2020 | CN | 112512471 | A | |

Form PCT/ISA/210 (patent family annex) (July 2022)

48

**EP 4 710 903 A1**

**REFERENCES CITED IN THE DESCRIPTION**

**Patent documents cited in the description**

- JP 2020022585 A
- JP 2023083677 A